# EUROPEAN PATENT SPECIFICATION

(11) **EP 2 462 199 B1**
(45) Date of publication and mention of the grant of the patent: **20.05.2015**
(21) Application number: 09788029.8
(22) Date of filing: 04.08.2009
(51) Int. Cl.: A61Q 1/02, A61Q 1/06, A61Q 1/12, A61Q 17/04, A61K 8/891, A61K 8/02, C09C 3/06, C09C 3/08, A61K 8/29

(54) **COMPOSITE PIGMENT AND METHOD FOR PREPARATION THEREOF**
VERBUNDPIGMENT UND VERFAHREN ZU SEINER HERSTELLUNG
PIGMENT COMPOSITE ET PROCÉDÉ DE PRÉPARATION DE CELUI-CI

(43) Date of publication of application: 13.06.2012
(73) Proprietor: L'Oréal, 75008 Paris (FR)
(72) Inventor: SUZUKI, Takahiro, Kawasaki-shi Kanagawa 213-0012 (JP); KASAI, Takehiko, Kawasaki-shi Kanagawa 213-0012 (JP); MATSUFUJI, Shinichi, Kawasaki-shi Kanagawa 213-0012 (JP)
(74) Representative: Kling, Simone
(86) International application number: PCT/JP2009/064208
(87) International publication number: WO 2011/016144

(56) References cited:
- EP-A1- 0 753 295
- EP-A1- 1 579 849
- EP-A2- 0 787 776
- WO-A1-93/04660
- WO-A1-2005/011622

## Description

### TECHNICAL FIELD

The present invention relates to a composite pigment comprising a core particle in the form of a bowl, and UV filter(s) and/or coloring pigment(s), as well as a method for preparing the composite pigment.

### BACKGROUND ART

In accordance with the variety of needs in cosmetics, various research and developments have been performed. In particular, for powders for cosmetics, many types of surface treatments or composite powders have been proposed.

For example, JP-A-S63-86760 discloses composite pigments comprising a core sphere particle and fine particles covering the core particle.

However, these composite pigments have a poor feeling on use, and the UV filtering property deteriorates if fine UV filter particles are used to cover a core particle as compared to a simple mixture of the fine UV filter particles and the core particle.

The PCT application WO 93/04660 discloses pigment-coated particles for use in cosmetic compositions, which are hollow microspheres.

The European application EP-A-787776 discloses a composite pigment composed of TiO2 and a particular organic polymer. The organic polymer particle disclosed therein may be hollow.

The PCT application WO 2005/11622 discloses core particles which are porous.

### DISCLOSURE OF INVENTION

Thus, an objective of the present invention is to provide a novel composite pigment which can provide a better feeling on use without deteriorating the UV filtering property when fine UV filter particles are used for covering a core particle to form the composite pigment.

The above objective of the present invention can be achieved by a composite pigment comprising a substrate with a general concave shape, said substrate being at least in part covered by at least one layer comprising at least one UV filter and/or at least one coloring pigment.

The substrate with a general concave shape may have a mean diameter ranging from 0.1 µm to 30 µm.

The substrate may define an inner concave surface and an outer convex surface, opposite said inner concave surface, with the at least one layer essentially covering said outer convex surface or at least one layer essentially covering said inner concave surface. An intermediate layer may be present between said at least one layer and said inner concave surface.

The substrate with a general concave shape may be in the form of a portion of a hollow sphere.

The substrate with a general concave shape comprises poly(meth)acrylate, an organosilicone material, or mixtures thereof.

The substrate with a general concave shape defines an inner concave surface defining an inner peripheral edge and an outer convex surface having an outer peripheral edge, wherein the average diameter of said inner peripheral edge ranges from 0.1 to 20 µm, the average diameter of said outer peripheral edge ranges from 0.2 to 30 µm, and the average concavity of the inner concave surface, as measured along an axis perpendicular to said inner peripheral edge, ranges from 0.1 to 20 µm.

The coloring pigment is chosen from titanium dioxide, zirconium oxide, cerium oxide, zinc oxide, iron oxide, chromium oxide, manganese violet, ultramarine blue, chromium hydrate, ferric blue, aluminum powder, copper powder, carbon black, pigments of D&C type, lakes, pearlescent pigments, and mixtures thereof.

The UV filter may be organic or inorganic. The UV filter comprises an organic filter selected from the group consisting of anthranilic derivatives; dibenzoylmethane derivatives; cinnamic derivatives; salicylic derivatives; camphor derivatives; benzophenone derivatives; β,β-diphenylacrylate derivatives; triazine derivatives; benzotriazole derivatives; benzalmalonate derivatives; benzimidazole derivatives; imidazoline derivatives; bis-benzoazolyl derivatives; p-aminobenzoic acid (PABA) and derivatives thereof; methylenebis(hydroxyphenylbenzotriazole)derivatives; benzoxazole derivatives; screening polymers and screening silicones; dimers derived from α-alkylstyrene; 4,4-diarylbutadiene derivatives; octocrylene and derivatives thereof, guaiazulene and derivatives thereof, rutin and derivatives thereof, flavonoids, biflavonoids, oryzanol and derivatives thereof, quinic acid and derivatives thereof, phenols, retinol, cysteine, aromatic amino acid, peptides having an aromatic amino acid residue, and mixtures thereof or an inorganic UV filter selected from the group consisting of silicon carbide, metal oxides which may or may not be coated, and mixtures thereof.

In the composite pigment according to the present invention, the weight ratio of said substrate with a general concave shape to the UV filter(s) and/or coloring pigment(s) may be 100:1 to 100:500.

The composite pigment according to the present invention can be prepared by a method comprising a step of subjecting a substrate with a general concave shape and at least one UV filter and/or at least one coloring pigment to a hybridizer process.

The composite pigment according to the present invention can be contained in a cosmetic composition.

### BRIEF DESCRIPTION OF DRAWINGS

Fig. 1 is a cross sectional view of a substrate having the shape of a bowl.
Fig. 2 shows SEM images of a composite pigment according to Example 1. Fig. 2(a) shows an outer appearance of the composite pigment, and Fig. 2(b) shows a cross sectional view of the composite pigment.
Fig. 3 shows SEM images of a composite pigment according to Comparative Example 1. Fig. 3(a) shows an outer appearance of the composite pigment, and Fig. 3(b) shows a cross sectional view of the composite pigment.
Fig. 4 shows SEM images of a composite pigment according to Comparative Example 2. Fig. 4(a) shows an outer appearance of the composite pigment, and Fig. 4(b) shows a cross sectional view of the composite pigment.
Fig. 5 shows SEM images of a composite pigment according to Comparative Example 3. Fig. 5(a) shows an outer appearance of the composite pigment, and Fig. 5(b) shows a cross sectional view of the composite pigment.
Fig. 6 shows SEM images of a composite pigment according to Example 6. Fig. 6(a) shows an outer appearance of the composite pigment, and Fig. 6(b) shows a cross sectional view of the composite pigment.
Fig. 7 shows an SEM image of the composite pigment according to Example 7.
Fig. 8 shows an SEM image of a mixture of components corresponding to the composite pigment according to Example 7.

### BEST MODE FOR CARRYING OUT THE INVENTION

After diligent research, the inventors have discovered that it is possible to obtain a new composite pigment providing a better feeling on use without deteriorating the UV filtering property when fine UV filter particles are used for covering a core particle to form the composite pigment.

The new composite pigment according to the present invention comprises a substrate with a general concave shape being at least in part covered by at least one layer comprising at least one UV filter and/or at least one coloring pigment. The substrate and the layer function as a core and a coating, respectively, of the composite pigment.

Since the substrate has a general concave shape, the composite pigment including the substrate has a good feeling on use.

In addition, since the substrate having a general concave shape has a larger surface area and a complex light path as compared to a sphere, high scattering effects of UV rays can be obtained.

Further, the composite pigment according to the present invention can effectively camouflage defects of the skin relief and color, such as pores, blots and stains on the skin, due to the use of the substrate having a general concave shape.

Furthermore, since the UV filter(s) and/or coloring pigment(s) are firmly fixed on the substrate, it is possible for the composite pigment according to the present invention to reduce free UV filters and/or coloring pigment(s) which have a high friction coefficient such that they do not easily spread on the skin so that an unpleasant feeling on use is imparted to the skin. In addition, the UV filters and/or coloring pigment(s) cannot detach from the composite pigment to penetrate into the skin via pores on the skin, which may impart adverse effects on the skin because the barrier property of the skin is not strong in pores.

In particular, the substrate having a general concave shape tends to accumulate in pores on the skin, therefore, the composite pigment according to the present invention can protect pores on the skin, and can be easily removed by cleansing agents.

Hereafter, each of the elements constituting the composite pigment according to the present invention will be described in a detailed manner.

### (Substrate)

The substrate has a general concave shape. In other words, the substrate has at least one concavity. It is preferable that the substrate is in the form of a particle having at least one concavity, which may be referred to as a concave particle hereafter. The concave is not a small dimple or pit, but a large hollow or crater which preferably includes a geometrical center or a center of gravity of the particle. This concave particle preferably has a mean diameter ranging from 0.1 µm to 30 µm, preferably 0.1 µm to 20 µm, and more preferably 0.1 µm to 10 µm.

Preferably, the substrate defines an inner concave surface and an outer convex surface which is opposite to the inner concave surface. In particular, the substrate is preferably in the form of a portion of a hollow sphere or a bowl. The substrate may have a transverse cross section with the shape of a horseshoe or arch.

The substrate with a general concave shape defines an inner concave surface defining an inner peripheral edge and an outer convex surface having an outer peripheral edge, wherein the average diameter of said inner peripheral edge ranges from 0.1 to 20 µm, preferably 0.1 to 10 µm, the average diameter of the outer peripheral edge ranges from 0.2 to 30 µm, preferably 0.2 to 20 µm, and the average concavity of the inner concave surface, as measured along an axis perpendicular to said inner peripheral edge, ranges from 0.1 to 20 µm, preferably 0.1 to 10 µm.

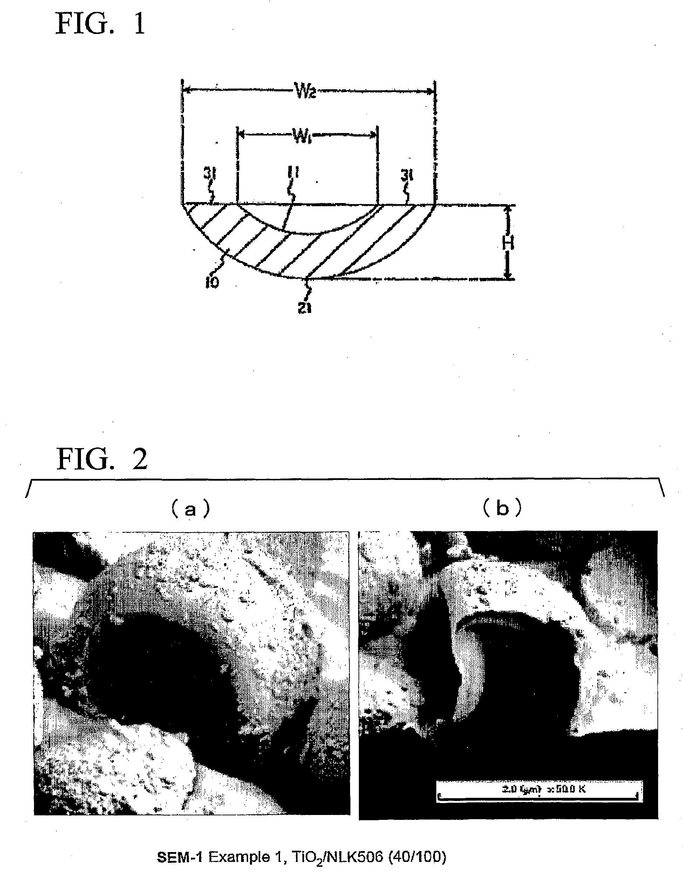

Preferably, the substrate is in the shape of a bowl formed (in longitudinal cross section), as shown in Figure 1, of a small inner concave surface (11) in the shape of an arc, of an outer convex surface (21) in the shape of an arc, and of segments (31) each of which connects the inner peripheral edge and the outer peripheral edge, the width (W1) between the inner peripheral edges ranging from 0.01 to 8 µm, preferably from 0.02 to 6 µm, on average, the width (W2) between the outer peripheral edges ranging from 0.05 to 10 µm, preferably from 0.06 to 8 µm, on average, and the height (H) of the outer convex surface (21) ranging from 0.015 to 8 µm, preferably from 0.03 to 6 µm, on average.

The dimensions mentioned above are obtained by calculating the mean of the dimensions of one hundred substrates chosen on an image obtained with a scanning electron microscope.

The substrate comprisespoly(meth)acrylate, an organosilicones or mixtures thereof.

The poly(meth)acrylates used as the material for the substrate are not limited as long as they are polymers or copolymers of a (meth)acrylate monomer.

Among the monomers capable of being employed in forming the poly(meth)acrylates, non-limiting mention may be made of:
(i) esters of acrylic acid of formula CH₂=CHCOOR₁ where R₁ is (a) a saturated or unsaturated, and linear or branched carbon chain, such as a C₂-C₁₂ hydrocarbon (alkyl)chain, optionally comprising at least one heteroatom chosen from O, N, S; and/or optionally substituted by at least one substituent chosen from -OH and halogen atoms (Cl, Br, I and F); or else R₁ is (b) a polyoxyethylene group comprising from 5 to 30 ethylene oxide units; or else R₁ is (c) a -R-(OC₂H₄)ₙ-H group with R = C₁-C₁₂ alkylene and n is an integer from 5 to 30 inclusive, among which non-limiting mention may thus be made of ethyl, propyl, n-butyl, isobutyl, 2-ethylhexyl, octyl, isooctyl, isodecyl, decyl, lauryl, tridecyl, hydroxyethyl and hydroxypropyl acrylates; and
(ii) esters of methacrylic acid of formula CH₂=C(CH₃)COOR₂ where R₂ is (a) a saturated or unsaturated, and linear or branched carbon chain, such as a C₂-C₁₂ hydrocarbon (alkyl) chain, optionally comprising at least one heteroatom chosen from O, N, S; and/or optionally substituted by at least one substituent chosen from -OH and halogen atoms (Cl, Br, I and F); or else R₂ is (b) a polyoxyethylene group comprising from 5 to 30 ethylene oxide units; or else R₂ is (c) a -R-(OC₂H₄)ₙ-H group with R = C₁-C₃₀ alkylene and n is an integer from 5 to 30 inclusive, among which non-limiting mention may thus be made of ethyl, propyl, n-butyl, isobutyl, 2-ethylhexyl, octyl, isooctyl, decyl, isodecyl, dodecyl, lauryl, tridecyl, myristyl, cetyl, palmityl, stearyl, behenyl and oleyl methacrylates.

The poly(meth)acrylates used as the material for the substrate may comprise at least one additional monomer. The additional monomer may be selected from the following:
(iii) vinyl esters of formula CH₂=CH-OCO-R₃ where R₃ is a saturated or unsaturated, and linear or branched carbon chain, for instance a C₂-C₁₂ hydrocarbon chain, among which non-limiting mention may be made of vinyl butyrate (or butanoate), vinyl ethylhexanoate, vinyl neononanoate and vinyl neododecanoate;
(iv) vinyl ethers of formula CH₂=CHOR₄ where R₄ is a saturated or unsaturated and linear or branched carbon chain, for instance a C₁-C₁₂ hydrocarbon chain, among which non-limiting mention may be made of vinyl ether, methyl vinyl ether, ethyl vinyl ether, ethylhexyl vinyl ether and butyl vinyl ether;
(v) N-alkyl(meth)acrylamides of formula CH₂=CHCONR₅R'₅ or CH₂=C(CH₃)CONR₅R'₅ where R₅ and R'₅ are, independently of one another, a hydrogen atom or a saturated or unsaturated and linear, cyclic or branched carbon chain, such as a C₆-C₂₈ hydrocarbon chain, optionally aromatic chain (aryl, aralkyl or alkylaryl), optionally comprising at least one heteroatom chosen from O, N, S; and/or optionally substituted by at least one substituent chosen from -OH and halogen atoms (Cl, Br, I and F); given that at least one of the R₅ and R'₅ radicals is other than hydrogen, among which non-limiting mention may be made of N-octylacrylamide and N-octadecylacrylamide;
(vi) vinyl compounds of formula CH₂=CHR₆ in which R₆ is a hydroxyl group; a C₁ to C₂₅ linear or branched alkyl group in which at least one heteroatom chosen from O, N, S and P is optionally inserted; it being possible for said alkyl group in addition to be optionally substituted by at least one substituent chosen from -OH and halogen atoms (Cl, Br, I-and F) ; a C₃ to C₈ cycloalkyl group, such as cyclohexyl, a C₆ to C₂₀ aryl group, such as phenyl, a C₇ to C₃₀ aralkyl group (C₁ to C₄ alkyl group), such as 2-phenylethyl or benzyl; a 4- to 12-membered heterocyclic group comprising at least one heteroatom chosen from O, N and S; a heterocycloalkyl group (alkyl of 1 to 4 carbons), such as furfuryl, furfurylmethyl or tetrahydrofurfurylmethyl, it being possible for said cycloalkyl, aryl, aralkyl, heterocyclic or heterocycloalkyl groups to be optionally substituted by at least one substituent chosen from the hydroxyl group, halogen atoms and linear or branched C₁-C₄ alkyl groups in which is optionally inserted at least one heteroatom chosen from O, N, S and P, it being possible for said alkyl groups in addition to be optionally substituted by at least one substituent chosen from -OH and halogen atoms (Cl, Br, I and F), among which examples of vinyl monomers include but are not limited to vinylcyclohexane, styrene and vinyl acetate;
(vii) acrylates of formula CH₂=CHCOOR₇ in which R₇ is a tert-butyl group; a C₃ to C₈ cycloalkyl group; a C₆ to C₂₀ aryl group; a C₇ to C₃₀ aralkyl group (C₁ to C₄ alkyl group); a 4- to 12-membered heterocyclic group comprising at least one heteroatom chosen from O, N and S; a heterocycloalkyl group (C₁ to C₄ alkyl), such as a furfuryl group; it being possible for the said cycloalkyl, aryl, aralkyl, heterocyclic or heterocycloalkyl groups to be optionally substituted by at least one substituent chosen from the hydroxyl group, halogen atoms and linear or branched C_{1-C4} alkyl groups in which is optionally inserted at least one heteroatom chosen from 0, N, S and P, it being possible for the said alkyl groups in addition to be optionally substituted by at least one substituent chosen from the hydroxyl group and halogen atoms (Cl, Br, I and F), among which examples of such monomers include but are not limited to tert-butyl, t-butylcyclohexyl, t-butylbenzyl, furfuryl and isobornyl acrylates, for instance;
(viii) methacrylates of formula CH₂=C(CH₃)COOR₈ in which R₈ is a linear or branched carbon group, for instance a C₁-C₆ hydrocarbon (alkyl) group, such as a methyl, ethyl, propyl or isobutyl group, it being possible for the said alkyl group in addition to be optionally substituted by at least one substituent chosen from OH and halogen atoms (Cl, Br, I and F); a C₃ to C₈ cycloalkyl group; a C₆ to C₂₀ aryl group; a C₇ to C₃₀ aralkyl group (C₁ to C₄ alkyl group); a 4- to 12-membered heterocyclic group comprising at least one heteroatom chosen from O, N and S; a heterocycloalkyl group (C₁-C₄ alkyl), such as a furfuryl group; it being possible for the said cycloalkyl, aryl, aralkyl, heterocyclic or heterocycloalkyl groups to be optionally substituted by at least one substituent chosen from OH, halogen atoms and linear or branched C₁-C₄ alkyl groups in which is optionally inserted at least one heteroatom chosen from O, N, S and P, it being possible for the said alkyl groups in addition to be optionally substituted by at least one substituent chosen from hydroxyl groups and halogen atoms (Cl, Br, I and F), among which examples of such monomers include but are not limited to methyl, ethyl, propyl, n-butyl, isobutyl, t-butylcyclohexyl, t-butylbenzyl, methoxyethyl, methoxypropyl and isobornyl methacrylates; and
(ix) (meth)acrylamides of formula CH₂=CHCONR₉R'₉ or CH₂=C(CH₃)CONR₉R'₉ in which R₉ and R'₉, which are identical or different, represent a hydrogen atom or a linear or branched C₁-C₅ alkyl group, such as an n-butyl, t-butyl or isopropyl group, among which examples of such monomers include but are not limited to N-butyl(meth)acrylamide, N-isopropyl(meth)acrylamide, N,N-dimethyl(meth)acrylamide and N,N-dibutyl(meth)acrylamide.

The poly(meth)acrylates used as the material for the substrate may have a number-average molecular weight (Mn) ranging from 2,000 to 1,000,000, for example ranging from 3,000 to 800,000, and further still for example ranging from 4,000 to 500,000.

The organosilicone material used as the material for the substrate is not limited as long as it is in the class of organopolysiloxanes. The organosilicone material of the substrate can be a crosslinked polysiloxane with a three-dimensional structure.

In one embodiment of the present invention, the crosslinked polysiloxane with a three-dimensional structure comprises units of formula (I): SiO₂, and of formula (II): R¹SiO_{1.5}, wherein R¹ comprises an organic group having a carbon atom directly connected to the silicon atom. The organic group can be chosen from a reactive organic group and an unreactive organic group. Preferably, the organic group is an unreactive organic group.

The unreactive organic group can be a C₁-C₄ alkyl group, such as a methyl, ethyl, propyl or butyl group, or a phenyl group. Preferably, the unreactive organic group is a methyl group.

The reactive organic group can be chosen from an epoxy group, a (meth)acryloyloxy group, an alkenyl group, a mercaptoalkyl group, an aminoalkyl group, a haloalkyl group, a glyceroxy group, an ureido group and a cyano group. Preferably, the reactive organic group can be chosen from an epoxy group, a (meth)acryloyloxy group, an alkenyl group, a mercaptoalkyl group and an aminoalkyl group. The reactive organic group generally comprises from 2 to 6 carbon atoms, preferably from 2 to 4 carbon atoms.

Among the epoxy groups that can be used, non-limiting mention may be made of a 2-glycidoxyethyl group, a 3-glycidoxypropyl group or a 2-(3,4-epoxycyclohexyl)propyl group.

Among the (meth)acryloyloxy groups that may be used, non-limiting mention may be made of a 3-methacryloyloxypropyl group or a 3-acryloyloxypropyl group.

Among the alkenyl groups that may be used, non-limiting mention may be made of a vinyl group, an allyl group or an isopropenyl group.

Among the mercaptoalkyl groups that may be used, non-limiting mention may be made of a mercaptopropyl group or a mercaptoethyl group.

Among the aminoalkyl groups that may be used, non-limiting mention may be made of a 3-[(2-aminoethyl)amino]propyl group, a 3-aminopropyl group or an N,N-dimethylaminopropyl group.

Among the haloalkyl groups that may be used, non-limiting mention may be made of a 3-chloropropyl group or a trifluoropropyl group.

Among the glyceroxy groups that may be used, non-limiting mention may be made of a 3-glyceroxypropyl group or a 2-glyceroxyethyl group.

Among the ureido groups that may be used, non-limiting mention may be made of a 2-ureidoethyl group.

Among the cyano groups that may be used, non-limiting mention may be made of a cyanopropyl group or a cyanoethyl group.

Preferably, in the unit of formula (II), R¹ denotes a methyl group.

In one embodiment of the present invention, the organosilicone material comprises the units (I) and (II) according to a unit (I)/unit (II) molar ratio ranging from 30/70 to 50/50. In a further embodiment of the present invention, the unit (I)/unit (II) ratio may range from 35/65 to 45/55.

The particles of the organosilicone material can be capable of being obtained according to a process comprising:
(a) introducing into an aqueous medium, in the presence of at least one hydrolysis catalyst and optionally of at least one surfactant, a compound (III) of formula SiX₄ and a compound (IV) of formula RSiY₃, wherein X and Y are chosen from, independently of one another, a C₁-C₄ alkoxy group, an alkoxyethoxy group including a C₁-C₄ alkoxy group, a C₂-C₄ acyloxy group, an N,N-dialkylamino group including a C₁-C₄ alkyl group, a hydroxyl group, a halogen atom and a hydrogen atom, and R is an organic group comprising a carbon atom connected directly to the silicon atom; and
(b) bringing the mixture resulting from stage (a) into contact with an aqueous solution including at least one polymerization catalyst and optionally at least one surfactant, at a temperature of between 30 and 85°C, for at least two hours.

Stage (a) corresponds to a hydrolysis reaction and stage (b) corresponds to a condensation reaction.

In stage (a), the molar ratio of the compound (III) to the compound (IV) generally ranges from 30/70 to 50/50. In one embodiment of the present invention, the molar ratio of compound (III) to compound (IV) ranges from 35/65 to 45/45.

In a further embodiment of the present invention, the molar ratio of compound (III) to compound (IV) is 40/60. The ratio by weight of the water to the total weight of the compounds (III) and (IV) can range from 10/90 to 70/30. The order of introduction of the compounds (III) and (IV) generally depends on their rate of hydrolysis. The temperature of the hydrolysis reaction generally ranges from 0 to 40°C and usually does not exceed 30°C in order to prevent premature condensation of the compounds.

For the X and Y groups of the compounds (III) and (IV), non-limiting mention may be made of the following groups:
C₁-C₄ alkoxy groups such as the methoxy or ethoxy groups; alkoxyethoxy groups including a C₁-C₄ alkoxy group, such as the methoxyethoxy or butoxyethoxy groups;
C₂-C₄ acyloxy groups such as the acetoxy or propionyloxy groups;
N,N-dialkylamino groups including a C₁-C₄ alkyl group, such as the dimethylamino or diethylamino groups; and
halogen atoms such as the chlorine or bromine atoms.

Among the compounds of formula (III) that may be used according to the present disclosure, non-limiting mention may be made of tetramethoxysilane, tetraethoxysilane, tetrabutoxysilane, trimethoxyethoxysilane, tributoxyethoxysilane, tetraacetoxysilane, tetrapropioxysilane, tetra(dimethylamino)silane, tetra(diethylamino)silane, silanetetraol, chlorosilanetriol, dichlorodisilanol, tetrachlorosilane or chlorotrihydrosilane. In one embodiment of the present invention, the compound of formula (III) is chosen from tetramethoxysilane, tetraethoxysilane, and tetrabutoxysilane, and mixtures thereof.

The compound of formula (III) results, after the polymerization reaction, in the formation of the units of formula (I).

The compound of formula (IV) results, after the polymerization reaction, in the formation of the units of formula (II).

The R group in the compound of formula (IV) has the meaning as described for the R¹ group for the compound of formula (II).

Among examples of compounds of formula (IV) comprising an unreactive organic group R, non-limiting mention may be made of methyltrimethoxysilane, ethyltriethoxysilane, pro.pyltributoxysilane, butyltributoxysilane, phenyltrimethoxyethoxysilane, methyltributoxyethoxysilane, methyltriacetoxysilane, methyltripropioxysilane, methyltri(dimethylamino)silane, methyltri(diethylamino)silane, methylsilanetriol, methylchlorodisilanol, methyltrichlorosilane or methyltrihydrosilane.

As examples of compounds of formula (IV) comprising a reactive organic group R, non-limiting mention may be made of:
silanes having an epoxy group, such as (3-glycidoxypropyl)trimethoxysilane, (3-glycidoxypropyl)triethoxysilane, [2-(3,4-epoxycyclohexyl)ethyl]trimethoxysilane, (3-glycidoxypropyl)methyldimethoxysilane, (2-glycidoxyethyl)methyldimethoxysilane, (3-glycidoxypropyl)dimethylmethoxysilane or (2-glycidoxyethyl)dimethylmethoxysilane;
silanes having a (meth)acryloyloxy group, such as (3-methacryloyloxypropyl)trimethoxysilane or (3-acryloyloxypropyl)trimethoxysilane;
silanes having an alkenyl group, such as vinyltrimethoxysilane, allyltrimethoxysilane or isopropenyltrimethoxysilane;
silanes having a mercapto group, such as mercaptopropyltrimethoxysilane or mercaptoethyltrimethoxysilane;
silanes having an aminoalkyl group, such as (3-aminopropyl)trimethoxysilane, (3-[(2-aminoethyl)amino]propyl)trimethoxysilane, (N, N-dimethylaminopropyl)trimethoxysilane or (N,N-dimethylaminoethyl)trimethoxysilane;
silanes having a haloalkyl group, such as (3-chloropropyl)trimethoxysilane or trifluoropropyltrimethoxysilane;
silanes having a glyceroxy group, such as (3-glyceroxypropyl)trimethoxysilane or di(3-glyceroxypropyl)dimethoxysilane;
silanes having a ureido group, such as (3-ureidopropyl)trimethoxysilane, (3-ureidopropyl)methyidimethoxysilane or (3-ureidopropyl)dimethylmethoxysilane; and
silanes having a cyano group, such as cyanopropyltrimethoxysilane, cyanopropylmethyldimethoxysilane or cyanopropyldimethylmethoxysilane.

In one embodiment of the present invention, the compound of formula (IV) comprising a reactive organic group R is chosen from silanes having an epoxy group, silanes having a (meth)acryloyloxy group, silanes having an alkenyl group, silanes having a mercapto group and silanes having an aminoalkyl group.

In another embodiment of the present invention, compounds (III) and (IV) can be tetraethoxysilane and methyltrimethoxysilane, respectively.

Use may independently be made, as hydrolysis and polymerization catalysts, of basic catalysts, such as sodium hydroxide, potassium hydroxide, sodium carbonate, sodium hydrogencarbonate or amines (such as ammonia, trimethylamine, triethylamine or tetramethylammonium hydroxide), or acidic catalysts chosen from organic acids, such as citric acid, acetic acid, methanesulphonic acid, p-toluenesulphonic acid, dodecylbenzenesulphonic acid or dodecylsulphonic acid, or inorganic acids, such as hydrochloric acid, sulphuric acid or phosphoric acid. When it is present, the surfactant used can be a nonionic or anionic surfactant or a mixture of the two. Sodium dodecyl-benzenesulphonate can be used as anionic surfactant. The end of the hydrolysis is marked by the disappearance of the products (III) and (IV), which are insoluble in water, and the production of a homogeneous liquid layer.

The condensation stage (b) can use the same catalyst as the hydrolysis stage or another catalyst chosen from those mentioned above.

At the conclusion of this process, a suspension in water of fine organosilicone particles is obtained, wherein the particles can optionally be separated subsequently from the medium. The process described above can thus comprise an additional stage of filtration, for example on a membrane filter, of the product resulting from stage (b), optionally followed by a stage of centrifuging the filtrate, intended to separate the particles from the liquid medium, and then by a stage of drying the particles. Other separation methods can, of course, be employed.

In one embodiment of the present invention, the particles obtained (or the spheres) have a mean diameter ranging from 0.1 to 30 µm.

The shape of the portions of hollow spheres obtained according to the above process and their dimensions will depend in particular on the method used to bring the products into contact in stage (b).

A somewhat basic pH and introduction under cold conditions of the polymerization catalyst into the mixture resulting from stage (a) will result in portions of hollow spheres with the shape of round-bottomed "bowls", whereas a somewhat acidic pH and dropwise introduction of the mixture resulting from stage (a) into the hot polymerization catalyst will result in portions of hollow spheres having a transverse cross section with the shape of a horseshoe.

The details of the preparation of the substrate are described in JP-A-2003-128788 which is incorporated herein by reference.

Among the concave particles which can be used according to the invention, non-limiting mention may be made of:
particles composed of the crosslinked organosilicone TAK-110 (crosslinked methylsilanol/silicate polymer) from Takemoto Oil & Fat, with the shape of a bowl, with a width of 2.5 µm, a height of 1.2 µm and a thickness of 150 nm (particles sold under the name NLK-506 by Takemoto Oil & Fat);
particles composed of the crosslinked organosilicone TAK-110 (crosslinked methylsilanol/silicate polymer) from Takemoto Oil & Fat, with the shape of a bowl, with a width of 2.5 µm, a height of 1.5 µm and a thickness of 350 nm;
particles composed of the crosslinked organosilicone TAK-110 (crosslinked methylsilanovsilicate polymer) from Takemoto Oil & Fat, with the shape of a bowl, with a width of 0.7 µm, a height of 0.35 µm and a thickness of 100 nm;
particles composed of the crosslinked organosilicone TAK-110 (crosslinked methylsilanol/silicate polymer) from Takemoto Oil & Fat, with the shape of a bowl, with a width of 7.5 µm, a height of 3.5 µm and a thickness of 200 nm; and
particles composed of the crosslinked organosilicone TAK-110 (crosslinked methylsilanol/silicate polymer) from Takemoto Oil & Fat, with the shape of a rugby-ball, with a particle size of about 3µm (particles sold under the name NLK-602 by Takemoto Oil & Fat).

### (Layer on Substrate)

The substrate is at least partially covered by at least one layer comprising at least one UV filter and/or at least one coloring pigment. The layer may be referred to as a coating layer. Preferably, 10% or more of the surface of the substrate is covered by the coating layer(s). More preferably, 50% or more of the surface is covered by the coating layer(s). More preferably, 80% or more of the substrate is covered by the coating layer(s). Most preferably, the entire surface of the substrate is covered by the coating layer(s).

If the substrate defines an inner concave surface and an outer convex surface which is opposite to the inner concave surface, the inner concave surface and/or the outer convex surface is/are covered by the coating layer(s).

It is possible for an intermediate layer to be present between the coating layer(s) and the substrate, in particular between the coating layer(s) and the inner concave surface. The intermediate layer may be formed by any material such as an organic substance. Alternatively, the intermediate layer may be an air space forming a hollow.

The thickness of the coating layer may vary depending on several factors such as the size of the substrate. Typically, the thickness of the coating layer may range from 0.001 µm to 20 µm, preferably 0.01 µm to 15 µm, and more preferably from 0.03 µm to 10 µm, and more preferably from 0.1 µm to 5 µm.

If there are two or more coating layers on the substrate, the thickness and the composition of the coating layers may be the same as or different from each other.

The coating layer(s) may comprise, other than the UV filter(s) and/or the coloring pigment(s), any additional material(s) such as a binder, preferably non-liquid binder. The additional material(s) may be present in an amount ranging from 1 to 50wt% relative to the total weight of the additional material(s), UV filter(s) and coloring pigment(s). However, it is preferable that the coating layer(s) consists of the UV filter(s) and/or the coloring pigment(s).

### (UV Filters)

The 'layer(s) covering the substrate include(s) one or more UV filters. The UV filter may be active in the UV-A and/or UV-B region. The UV filter may be hydrophilic and/or lipophilic and/or properly insoluble in solvents commonly used in cosmetics.

The UV filter may be in the form of a liquid or a solid such as a particle. If the UV filter is in the form of a solid particle, it is preferable that the primary particle diameter thereof ranges from 1 nm to 5 µm, preferably 10 nm to 1 µm, more preferably 10 nm to 100 nm, and more preferably 10 nm to 20 nm.

If UV filter(s) in the form of fine particles is/are used, the composite pigment according to the present invention has an effect in that it can provide not a white appearance but a transparent or clear appearance, because the fine particles do not aggregate but spread on the substrate. It should be noted that free fine particles of UV filter(s) easily aggregate to give a white appearance to the skin.

The UV filter may be organic or inorganic. If two or more UV filters are used, the material(s) of the UV filters may be the same as or different from each other.

The organic UV filter is selected from the group consisting of anthranilic derivatives; dibenzoylmethane derivatives; cinnamic derivatives; salicylic derivatives; camphor derivatives; benzophenone derivatives; β,β-diphenylacrylate derivatives; triazine derivatives; benzotriazole derivatives; benzalmalonate derivatives; benzimidazole derivatives; imidazoline derivatives; bis-benzoazolyl derivatives; p-aminobenzoic acid (PABA) and derivatives thereof; methylenebis(hydroxyphenylbenzotriazole) derivatives; benzoxazole derivatives; screening polymers and screening silicones; dimers derived from α-alkylstyrene; 4,4-diarylbutadienes; octocrylene and derivatives thereof, guaiazulene and derivatives thereof, rutin and derivatives thereof, flavonoids, biflavonoids, oryzanol and derivatives thereof, quinic acid and derivatives thereof, phenols, retinol, cysteine, aromatic amino acid, peptides having an aromatic amino acid residue, and mixtures thereof.

Mention may be made, as examples of organic UV filter, of those denoted below under their INCI names, and mixtures thereof.

Anthranilic derivatives: Menthyl anthranilate, marketed under the trademark "Neo Heliopan MA" by Haarmann and Reimer.

Dibenzoylmethane derivatives: Butyl methoxydibenzoylmethane, marketed in particular under the trademark "Parsol 1789" by Hoffmann-LaRoche; and Isopropyl dibenzoylmethane.

Cinnamic derivatives: Ethylhexyl methoxycinnamate, marketed in particular under the trademark "Parsol MCX" by Hoffmann-LaRoche; Isopropyl methoxycinnamate; Isopropoxy methoxycinnamate; Isoamyl methoxycinnamate, marketed under the trademark "Neo Heliopan E 1000" by Haarmann and Reimer; Cinoxate (2-ethoxyethyl-4-m6thoxy cinnamate); DEA Methoxycinnamate; Diisopropyl methylcinnamate; and Glyceryl ethylhexanoate dimethoxycinnamate.

Salicylic derivatives: Homosalate (homomentyl salicylate), marketed under the trademark "Eusolex HMS" by Rona/EM Industries; Ethylhexyl salicylate, marketed under the trademark "Neo Heliopan OS" by Haarmann and Reimer; Glycol salicylate; Butyloctyl salicylate; Phenyl salicylate; Dipropyleneglycol salicylate, marketed under the trademark. "Dipsal" by Scher; and TEA salicylate, marketed under the trademark "Neo Heliopan TS" by Haarmann and Reimer.

Camphor derivatives, in particular, benzylidenecamphor derivatives: 3-Benzylidene camphor, manufactured under the trademark "Mexoryl SD" by Chimex; 4-Methylbenzylidene camphor, marketed under the trademark "Eusolex 6300" by Merck; Benzylidene camphor sulfonic acid, manufactured under the trademark "Mexoryl SL" by Chimex; Camphor benzalkonium methosulfate, manufactured under the trademark "Mexoryl SO" by Chimex; Terephthalylidene dicamphor sulfonic acid, manufactured under the trademark "Mexoryl SX" by Chimex; and Polyacrylamidomethyl benzylidene camphor, manufactured under the trademark "Mexoryl SW" by Chimex.

Benzophenone derivatives: Benzophenone-1 (2,4-dihydroxybenzophenone), marketed under the trademark "Uvinul 400" by BASF; Benzophenone-2 (Tetrahydroxybenzophenone), marketed under the trademark "Uvinul D50" by BASF; Benzophenone-3(2-hydroxy-4-methoxybenzophenone) or Oxybenzone, marketed under the trademark "Uvinul M40" by BASF; Benzophenone-4(Hydroxymethoxy benzophonene sulfonic acid), marketed under the trademark "Uvinul MS40" by BASF; Benzophenone-5(Sodium hydroxymethoxy benzophenone Sulfonate); Benzophenone-6(Dihydroxy dimethoxy benzophenone); marketed under the trademark "Helisorb 11" by Norquay; Benzophenone-8, marketed under the trademark "Spectra-Sorb UV-24" by American Cyanamid; Benzophenone-9(Disodium dihydroxy dimethoxy benzophenonedisulfonate), marketed under the trademark "Uvinul DS-49" by BASF; Benzophenone-12, and n-Hexyl 2-(4-diethylamino-2-hydroxybenzoyl)benzoate.

β,β-Diphenylacrylate derivatives: Octocrylene, marketed in particular under the trademark "Uvinul N539" by BASF; and Etocrylene, marketed in particular under the trademark "Uvinul N35" by BASF.

Triazine derivatives: bis-Ethylhexyloxyphenol methoxyphenyl triazine, marketed under the trademark "Tinosorb S" by Ciba-Geigy; Ethylhexyl triazone, marketed in particular under the trademark "Uvinul T150" by BASF; Diethylhexyl butamido triazone, marketed under the trademark "Uvasorb HEB" by Sigma 3V; 2,4,6-Tris(dineopentyl 4'-aminobenzalmalonate)-s-triazine; and the symmetrical triazine screening agents described in U.S. Pat. No. 6,225,467, WO 2004/085412 (see compounds 6 and 9) or the document "Symmetrical Triazine Derivatives", IP.COM Journal, IP.COM INC, WEST HENRIETTA, NY, US (20 Sep. 2004), in particular the 2,4,6-tris(biphenyl)-1,3,5-triazines (especially 2,4,6-tris(biphenyl-4-yl)-1,3,5-triazine) and 2,4,6-tris(terphenyl)-1,3,5-triazine, which is taken up again in WO 06/035000, WO 06/034982, WO 06/034991, WO 06/035007, WO 2006/034992 and WO 2006/034985.

Benzotriazole derivatives, in particular, phenylbenzotriazole derivatives: Drometrizole trisiloxane, marketed under the trademark "Silatrizole" by Rhodia Chimie or "Mexoryl XL" by L'Oreal; 2-(2,4-dihydroxyphenyl)-2H-benzotriazole; 2-(2-hydroxy-5-tert-butylphenyl)-2H-benzotriazole; 2-(2-hydroxyphenyl)-2H-benzotriazole.

Benzalmalonate derivatives: Dineopentyl 4'-methoxybenzalmalonate, and polyorganosiloxane comprising benzalmalonate functional groups, such as Polysilicone-15, marketed under the trademark "Parsol SLX" by Hoffmann-LaRoche.

Benzimidazole derivatives, in particular, phenylbenzimidazole derivatives: Phenylbenzimidazole sulfonic acid, marketed in particular under the trademark "Eusolex 232" by Merck, and Disodium phenyl dibenzimidazole tetrasulfonate, marketed under the trademark "Neo Heliopan AP" by Haarmann and Reimer.

Imidazoline derivatives: Ethylhexyl dimethoxybenzylidene dioxoimidazoline propionate.

bis-Benzoazolyl derivatives: The derivatives as described in EP-669,323 and U.S. Pat. No. 2,463,264.

para-Aminobenzoic acid and derivatives thereof: PABA (p-Aminobenzoic acid), Ethyl PABA, Ethyl dihydroxypropyl PABA, Penthyl dimethyl PABA, Ethylhexyl dimethyl PABA, marketed in particular under the trademark "Escalol 507" by ISP, Glyceryl PABA, and PEG-25 PABA, marketed under the trademark "Uvinul P25" by BASF.

Methylenebis(hydroxyphenylbenzotriazole) derivatives: Methylene bis-benzotriazolyl tetramethylbutylphenol, marketed in the solid form under the trademark "Mixxim BB/100" by Fairmount Chemical or in the micronized form in aqueous dispersion under the trademark "Tinosorb M" by Ciba Specialty Chemicals, and the derivatives as described in U.S. Pat. Nos. 5,237,071, 5,166,355, GB-2,303,549, DE-197,26,184 and EP-893,119.

Benzoxazole derivatives: 2,4-bis[5-1(dimethylpropyl)benzoxazol-2-yl-(4-phenyl)imino]-6-(2-ethylhexyl)imino'-1,3,5-triazine, marketed under the trademark of Uvasorb K2A by Sigma 3V.

Screening polymers and screening silicones: The silicones described in WO 93/04665.

Dimers derived from α-alkylstyrene: The dimers described in DE-19855649.

4,4-Diarylbutadiene Derivatives: 1,1-Dicarboxy(2,2'-dimethylpropyl)-4,4-diphenylbutadiene.

Octocrylene and derivatives thereof: Octocrylene.

Quaiazulene and derivatives thereof: Guaiazulene, and Sodium Guaiazulene Sulfonate.

Rutin and derivatives thereof: Rutin, and Glucosylrutin.

Flavonoids: Robustin (isoflavonoid), Genistein (flavonoid), Tectochrysin (flavonoid), and Hispidone (flavonoid).

Biflavonoids: Lanceolatin A, Lanceolatin B, and Hypnumbiflavonoid A.

Oryzanol and derivatives thereof: Γ-oryzanol.

Quinic acid and derivatives thereof: Quinic acid.

Phenols: Phenol.

Retinols: Retinol.

Cysteines: L-Cysteine.

Peptides having an aromatic amino acid residue: Peptides having tryptophan, tyrosine or phenylalanine.

The preferred organic UV screening agents are selected from: Ethylhexyl methoxycinnamate, Homosalate, ethylhexyl salicylate, octocrylene, phenylbenzimidazole sulfonic acid, Benzophenone-3, Benzophenone-4, Benzophenone-5, n-hexyl 2-(4-diethylamino-2-hydroxybenzoyl)benzoate, 4-methylbenzylidene camphor, terephthalylidene dicamphor sulfonic acid, disodium phenyl dibenzimidazole tetrasulfonate, ethylhexyl triazone, bis-ethylhexyloxyphenol methoxyphenyl triazine, diethylhexyl butamido triazone, 2,4,6-tris(dineopentyl 4'-aminobenzalmalonate)-s-triazine, 2,4,6-tris(diisobutyl 4'-aminobenzalmalonate)-s-triazine, 2,4,6-tris(biphenyl-4-yl)-1,3,5-triazine, 2,4,6-Tris(terphenyl)-1,3,5-triazine, methylene bis-Benzotriazolyl tetramethylbutylphenol, Drometrizole trisiloxane, Polysilicone-15, dineopentyl 4'-methoxybenzalmalonate, 1,1-dicarboxy(2,2'-dimethylpropyl)-4,4-diphenylbutadiene, 2,4-Bis[5-1 (dimethylpropyl)benzoxazol-2-yl-(4-phenyl)imino]-6-(2-ethylhexyl)imino-1,3,5-triazine, and their mixtures.

More preferable organic UV filters are ethylhexyl methoxycinnamate, Drometrizole trisiloxane, and mixtures thereof.

The inorganic UV filter is selected from the group consisting of silicon carbide, metal oxides which may or may not be coated, and mixtures thereof.

Preferably, the inorganic UV filters are selected from pigments (mean size of the primary particles: generally from 5 nm and 100 nm, preferably from 10 nm and 50 nm) formed of metal oxides which may or may not be coated, such as, for example, pigments formed of titanium oxide (amorphous or crystalline in the rutile and/or anatase form), iron oxide, zinc oxide, zirconium oxide or cerium oxide, which are all UV photoprotective agents well known per se.

The pigments may or may not be coated. The coated pigments are pigments which have been subjected to one or more surface treatments of chemical, electronic, mechanochemical and/or mechanical nature with compounds such as described, for example, in Cosmetics & Toiletries, February 1990, Vol. 105, pp. 53-64, such as amino acids, beeswax, fatty acids, fatty alcohols, anionic surfactants, lecithins, sodium, potassium, zinc, iron or aluminum salts of fatty acids, metal alkoxides (titanium or aluminum alkoxides), polyethylene, silicones, proteins (collagen, elastin), alkanolamines, silicon oxides, metal oxides or sodium hexametaphosphate.

In a known manner, the silicones are organosilicon polymers or oligomers comprising a linear or cyclic and branched or crosslinked structure, of variable molecular weights, obtained by polymerization and/or polycondensation of suitable functional silanes and essentially composed of a repetition of main units in which the silicon atoms are connected to one another via oxygen atoms (siloxane bond), optionally substituted hydrocarbon radicals being connected directly to the said silicon atoms via a carbon atom.

The term "silicones" also encompasses the silanes necessary for their preparation, in particular alkylsilanes.

The silicones used for the coating of the pigments suitable for the present invention are preferably selected from the group consisting of alkylsilanes, polydialkylsiloxanes and polyalkylhydrosiloxanes. More preferably still, the silicones are selected from the group consisting of octyltrimethylsilane, polydimethylsiloxanes and polymethylhydrosiloxanes.

Of course, the pigments formed of metal oxides may, before their treatment with silicones, have been treated with other surfacing agents, in particular with cerium oxide, alumina, silica, aluminum compounds, silicon compounds or their mixtures.

The coated pigments are more particularly titanium oxides coated:
with silica, such as the product "Sunveil" from Ikeda,
with silica and with iron oxide, such as the product "Sunveil F" from Ikeda,
with silica and with alumina, such as the products "Microtitanium Dioxide MT 500 SA" and "Microtitanium Dioxide MT 100 SA" from Tayca, "Tioveil" from Tioxide and "Mirasun TiW 60" from Rhodia,
with alumina, such as the products "Tipaque TTO-55 (B)" and "Tipaque TTO-55 (A)" from Ishihara and "UVT 14/4" from Kemira, with alumina and with aluminum stearate, such as the product "Microtitanium Dioxide MT 100 T, MT 100 TX, MT 100 Z or MT-01" from Tayca, the products "Solaveil CT-10 W" and "Solaveil CT 100" from Uniqema and the product "Eusolex T-AVO" from Merck, with silica, with alumina and with alginic acid, such as the product "MT-100 AQ" from Tayca,
with aluminum stearate, such as the product "MT-100 TV" from Tayca, primary particle diameter is 15 nm,
with alumina and with aluminum laurate, such as the product "Microtitanium Dioxide MT 100 S" from Tayca,
with iron oxide and with iron stearate, such as the product "Microtitanium Dioxide MT 100 F" from Tayca,
with zinc oxide and with zinc stearate, such as the product "BR351" from Tayca,
with silica and with alumina and treated with a silicone, such as the products "Microtitanium Dioxide MT 600 SAS", "Microtitanium Dioxide MT 500 SAS" and "Microtitanium Dioxide MT 100 SAS" from Tayca,
with silica, with alumina and with aluminum stearate and treated with a silicone, such as the product "STT-30-DS" from Titan Kogyo,
with silica and treated with a silicone, such as the product "UV-Titan X 195" from Kemira,
with alumina and treated with a silicone, such as the products "Tipaque TTO-55 (S)" from Ishihara or "UV Titan M 262" from Kemira,
with triethanolamine, such as the product "STT-65-S" from Titan Kogyo,
with stearic acid, such as the product "Tipaque TTO-55 (C)" from Ishihara, or
with sodium hexametaphosphate, such as the product "Microtitanium Dioxide MT 150 W" from Tayca.

Other titanium oxide pigments treated with a silicone are preferably TiO2 treated with octyltrimethylsilane and for which the mean size of the individual particles is from 25 and 40 nm, such as that marketed under the trademark "T 805" by Degussa Silices, TiO2 treated with a polydimethylsiloxane and for which the mean size of the individual particles is 21 nm, such as that marketed under the trademark "70250 Cardre UF TiO2SI3" by Cardre, anatase/rutile TiO2 treated with a polydimethylhydrosiloxane and for which the mean size of the individual particles is 25 nm, such as that marketed under the trademark "Microtitanium Dioxide USP Grade Hydrophobic" by Color Techniques.

The uncoated titanium oxide pigments are, for example, marketed by Tayca under the trademarks "Microtitanium Dioxide MT500B" or "Microtitanium Dioxide MT600B", by Degussa under the trademark "P 25", by Wacker under the trademark "Oxyde de titane transparent PW", by Miyoshi Kasei under the trademark "UFTR", by Tomen under the trademark "ITS" and by Tioxide under the trademark "Tioveil AQ".

The uncoated zinc oxide pigments are, for example:
those marketed under the trademark "Z-cote" by Sunsmart;
those marketed under the trademark "Nanox" by Elementis; and
those marketed under the trademark "Nanogard WCD 2025" by Nanophase Technologies.

The coated zinc oxide pigments are, for example:
those marketed under the trademark "Oxide Zinc CS-5" by Toshiba (ZnO coated with polymethylhydrosiloxane);
those marketed under the trademark "Nanogard Zinc Oxide FN" by Nanophase Technologies (as a 40% dispersion in Finsolv TN, C₁₂-C₁₅ alkyl benzoate);
those marketed under the trademark "Daitopersion Zn-30" and "Daitopersion Zn-50" by Daito (dispersions in oxyethylenated polydimethylsiloxane/cyclopolymethylsiloxane comprising 30% or 50% of zinc nanooxides coated with silica and polymethylhydrosiloxane);
those marketed under the trademark "NFD Ultrafine ZnO" by Daikin (ZnO coated with phosphate of perfluoroalkyl and copolymer based on perfluoroalkylethyl as a dispersion in cyclopentasiloxane);
those marketed under the trademark "SPD-Z1" by Shin-Etsu (ZnO coated with silicone-grafted acrylic polymer dispersed in cyclodimethylsiloxane);
those marketed under the trademark "Escalol Z100" by ISP (alumina-treated ZnO dispersed in the ethylhexyl methoxycinnamate/PVP-hexadecene copolymer/methicone mixture); and
those marketed under the trademark "Fuji ZnO-SMS-10" by Fuji Pigment (ZnO coated with silica and polymethylsilsesquioxane); those marketed under the trademark "Nanox Gel TN" by Elementis (ZnO dispersed at 55% in C₁₂-C₁₅ alkyl benzoate with hydroxystearic acid polycondensate).

The uncoated cerium oxide pigments are marketed, for example, under the trademark "Colloidal Cerium Oxide" by Rhone-Poulenc.

The uncoated iron oxide pigments are, for example, marketed by Arnaud under the trademarks "Nanogard WCD 2002 (FE 45B)", "Nanogard Iron FE 45 BL AQ", "Nanogard FE 45R AQ" or "Nanogard WCD 2006 (FE 45R)", or by Mitsubishi under the trademark "TY-220".

The coated iron oxide pigments are, for example, marketed by Arnaud under the trademarks "Nanogard WCD 2008 (FE 45B FN)", "Nanogard WCD 2009 (FE 45B 556)", "Nanogard FE 45 BL 345" or "Nanogard FE 45 BL" or by BASF under the trademark "Oxyde de fer transparent".

Mention may also be made of mixtures of metal oxides, in particular of titanium dioxide and of cerium dioxide, including the mixture of equal weights of titanium dioxide coated with silica and of cerium dioxide coated with silica marketed by Ikeda under the trademark "Sunveil A", and also the mixture of titanium dioxide and of zinc dioxide coated with alumina, with silica and with silicone, such as the product "M 261" marketed by Kemira, or coated with alumina, with silica and with glycerol, such as the product "M 211" marketed by Kemira.

The coated pigments are preferable because the coating may function as a binder for fixing the pigments on or in the substrates. In particular, titanium oxide coated with aluminum stearate such as the product "MT-100 TV" from Tayca is preferable.

The UV filter(s) may be used in the composite pigment according to the present invention in proportions such that the weight ratio of said substrate with a general concave shape to the UV filter(s) is 100:1 to 100:500, preferably 100:5 to 100:400, more preferably 100:10 to 100:200, more preferably 100:10 to 100:100, more preferably 100:10 to 100:50, and more preferably 100:10 to 100:30.

### (Coloring Pigments)

The term "coloring pigments" should be understood as meaning white or colored, inorganic or organic particles of any shape which are insoluble and are intended to color the composition.

If coloring pigment(s) is/are used, the composite pigment according to the present invention has an effect in that it can provide a clearer appearance, because the coloring pigments do not aggregate but spread on the substrate. It should be noted that free coloring pigments easily aggregate to give a dark appearance to the skin.

The pigments can be white or colored, inorganic and/or organic.

The inorganic pigment may be chosen among titanium dioxide, optionally surface treated, zirconium or cerium oxide, as well as zinc, (black, yellow or red) iron or chromium oxide, manganese violet, ultramarine blue, chromium hydrate and ferric blue, or metal powders, such as aluminum powder or copper powder. The pigments can also be chosen from nanopigments formed of metal oxides, such as titanium dioxide, zinc oxide, iron oxide, zirconium oxide, and cerium oxide, and mixtures thereof. The term "nanopigments" is understood to mean pigments having a mean particle size ranging from 1 nm to 500 nm, such as particle sizes ranging from 10 nm to 100 nm.

Theorganic pigment may be chosen among carbon black, pigments of D&C type and lakes, such as lakes based on cochineal carmine and on barium, strontium, calcium or aluminum. For example, Red 202 (Calcium bis[2-(3-carboxy-2-hydroxynephthylazo)-5-methylbenzenesulfonate) may be used as the pigment of D&C type.

The coloring pigment is chosen from titanium dioxide, zirconium oxide, cerium oxide, zinc oxide, iron oxide, chromium oxide, manganese violet, ultramarine blue, chromium hydrate, ferric blue, aluminum powder, copper powder, carbon black, pigments of D&C type, lakes, pearlescent pigments, and mixtures thereof.

The term "pearlescent pigments" should be understood as meaning iridescent particles of any shape, such as particles produced by certain shellfish in their shells or else synthesized.

The pearlescent agents can be chosen from white pearlescent agents, such as mica covered with titanium dioxide or with bismuth oxychloride; colored pearlescent agents, such as titanium oxide-coated mica covered with iron oxide, titanium oxide-coated mica covered with ferric blue or chromium oxide, or titanium oxide-coated mica covered with an organic pigment of the above-mentioned type; and pearlescent agents based on bismuth oxychloride.

The coloring pigment(s) may be used in the composite pigment according to the present invention in proportions such that the weight ratio of said substrate with a general concave shape to the coloring pigment(s) is 100:1 to 100:500, preferably 100:5 to 100:400, more preferably 100:10 to 100:200, more preferably 100:10 to 100:100, more preferably 100:10 to 100:50, and more preferably 100:10 to 100:30.

### (Method for Preparing Composite Pigment)

The composite pigment according to the present invention can be prepared by subjecting a substrate with a general concave shape and UV filter(s) and/or coloring pigment(s) to a hybridizer process.

The hybridizer process was developed in the 1980s. The hybridizer process is a class of mechanochemical fusion processes in which strong mechanical power is applied to a plurality of particles to cause a mechanochemical reaction to form a composite particle.

According to the hybridizer process, the mechanical power is imparted by a high speed rotor which can have a diameter from 10 cm to 1 m, and can rotate at a speed of 1,000 rpm to 100,000 rpm. Therefore, the hybridizer process can be defined as a mechanochemical fusion process using such a high speed rotor. The hybridizer process is performed in air or under dry conditions. Thus, due to the high speed rotation of the rotor, high speed air flow may be generated near the rotor. However, some liquid materials may be subjected to the hybridizer process together with solid materials. The term "hybridizer process" has been used as a technical term.

The hybridizer process can be performed by using a hybridization system marketed by, for example, Nara Machinery in Japan, in which at least two types of particles, typically core particles and fine particles, are fed into a hybridizer equipped with a high speed rotor having a plurality of blades in a chamber under dry conditions, and the particles are dispersed in the chamber and mechanical and thermal energy (e.g., compression, friction and shear stress) are imparted to the particles for a relatively short period of time such as 1 to 10 minutes, preferably 1 to 5 minutes. As a result, one type of particles (e.g., fine particles) is embedded or fixed on the other type of particle (e.g., core particle) to form a composite particle. It is preferable that the particles have been subjected to electrostatic treatment(s) such as shaking to form an "ordered mixture" in which one type of particles is spread to cover the other type of particle. The hybridizer process can also be performed by using a theta composer marketed by Tokuju Corporation in Japan.

According to the present invention, the substrate having a general concave shape and the UV filter(s) and/or coloring pigment(s), as well as other optional component(s) if necessary, can be fed into such a hybridizer to form a composite pigment. The hybridizer process can be performed by using a rotor rotating at about 8,000 rpm (100 m/sec) for about 5 minutes.

If both UV filter(s) and coloring pigment(s) are used for the composite pigment according to the present invention, they can be used in proportions such that the weight ratio of the substrate to the UV filter(s) and coloring pigment(s) is 100:1 to 100:500, preferably 100:5 to 100:400, more preferably 100:10 to 100:200, more preferably 100:10 to 100:100, more preferably 100:10 to 100:50, and more preferably 100:10 to 100:30.

The hybridizer process enables to provide ordered array (e.g., uniform coverage) of the fine particles of the UV filter(s) and/or coloring pigment(s) on the core substrate particle and provides strong bonds at the surface of the core and a layer comprising the fine particles.

It should be noted that the hybridizer process is quite different from other processes using, for example, a beads mill and a jet mill. In fact, a beads mill causes pulverization or aggregation of core particles, and a jet mill causes pulverization of core particles and uniform coating of a core particle by fine particles.

If necessary, an additional process for further coating the composite pigment by additional UV filter(s) and/or coloring material(s) may be performed. As a result of this additional process, the composite pigment according to the present invention may be coated with a further layer comprising UV filter(s) and/or coloring material(s), preferably consisting of UV filter(s) and/or coloring material(s).

### (Cosmetic Composition)

The composite pigment, as described above, can be present in the composition according to the present invention in an amount ranging from 0.01% to 99% by weight, preferably 0.1% to 50% by weight, and more preferably 1% to 30% by weight, relative to the total weight of the composition.

Preferably, the composite pigment according to the present invention can be used in cosmetic compositions to be applied to keratin substances such as skin, hair, and nails, providing UV shielding effects and/or coloring effects, as well as camouflaging effects, because the composite pigment can exhibit a good feeling on use as well as good UV filtering effects with a transparent or clear appearance and/or good coloring effects such as a more transparent or clear coloring, as well as skin defect concealing effects, without the risk of affecting the keratin substances.

The cosmetic composition according to the present invention may further comprise a filler and an oil.

As used herein, the term "filler" should be understood as meaning colorless natural or synthetic particles of any shape which are insoluble in the medium of the composition, whatever the temperature at which the composition is manufactured. Thus, the filler is different from the coloring pigment as described above.

The fillers may be inorganic or organic and of any shape (for instance, platelet, spherical, and oblong shapes) and with any crystallographic form (for example, sheet, cubic, hexagonal, orthorhombic, and the like). Examples of suitable additional fillers include, but are not limited to, talc; mica; silica; kaolin; powders of polyamide such as Nylon®; poly-β-3-alanine powders; polyethylene powders; polyurethane powders, such as the powder formed of hexamethylene diisocyanate and trimethylol hexyllactone copolymer sold under the name Plastic Powder D-400 by Toshiki; the powders formed of tetrafluoroethylene polymers (Teflon®); lauroyllysine; starch; boron nitride; polymeric hollow microspheres, such as microspheres of poly(vinylidene cliloride)/acrylonitrile, for example Expancel® (Nobel Industrie), and microspheres of acrylic acid copolymers; silicone resin powders, for example, silsesquioxane powders (for instance, silicone resin powders disclosed in European Patent No. 0 293 795 and Tospearls® from Toshiba); poly(methyl methacrylate) particles; precipitated calcium carbonate; magnesium carbonate; basic magnesium carbonate; hydroxyapatite; hollow silica microspheres; glass microcapsules; ceramic microcapsules; metal soaps derived from organic carboxylic acids comprising from 8 to 22 carbon atoms, for example, from 12 to 18 carbon atoms, such as zinc stearate, magnesium stearate, lithium stearate, zinc laurate, and magnesium myristate; barium sulphate; and mixtures thereof.

The filler may be present in the composition in an amount ranging from 0.1% to 80% by weight, with respect to the total weight of the composition, for example, from 1% to 25% by weight, or from 3% to 15% by weight.

The term "oil" is understood to mean a fatty substance which is liquid at ambient temperature (25°C).

Use may be made, as oils which can be used in the composition of the invention, for example, of hydrocarbon oils of animal origin, such as perhydrosqualene (or squalane); hydrocarbon oils of vegetable origin, such as triglycerides of caprylic/capric acids, for example those marketed by Stearineries Dubois or those marketed under the trademarks Miglyol 810, 812 and 818 by Dynamit Nobel, or oils of vegetable origin, for example sunflower, maize, soybean, cucumber, grape seed, sesame, hazelnut, apricot, macadamia, arara, coriander, castor, avocado or jojoba oil or shea butter oil; synthetic oils; silicone oils, such as volatile or nonvolatile polymethylsiloxanes (PDMSs) comprising a linear or cyclic silicone chain which are liquid or paste at ambient temperature; fluorinated oils, such as those which are partially hydrocarbon and/or silicone, for example those described in JP-A-2-295912; ethers, such as dicaprylyl ether (CTFA name) ; and esters, such as benzoate C₁₂-C₁₅ fatty alcohols (Finsolv TN from Finetex); arylalkyl benzoate derivatives, such as 2-phenylethyl benzoate (X-Tend 226 from ISP); amidated oils, such as isopropyl N-lauroylsarcosinate (Eldew SL-205 from Ajinomoto), and their mixtures.

The oily phase can also comprise one or more fatty substances selected, for example, from fatty alcohols (cetyl alcohol, stearyl alcohol, cetearyl alcohol), fatty acids (stearic acid) or waxes (paraffin wax, polyethylene waxes, carnauba wax, beeswax). The oily phase can comprise lipophilic gelling agents, surfactants or also organic or inorganic particles.

The oily phase can preferably represent from 1 to 70% of oil by weight, with respect to the total weight of the composition.

The composition according to the present invention may further comprise at least one additional conventional cosmetic ingredient which may be chosen, for example, from hydrophilic or lipophilic gelling and/or thickening agents, surfactants, antioxidants, fragrances, preservatives, neutralizing agents, sunscreens, vitamins, moisturizing agents, self-tanning compounds, antiwrinkle active agents, emollients, hydrophilic or lipophilic active agents, agents for combating pollution and/or free radicals, sequestering agents, film-forming agents, dermo-decontracting active agents, soothing agents, agents which stimulate the synthesis of dermal or epidermal macromolecules and/or which prevent their decomposition, antiglycation agents, agents which combat irritation, desquamating agents, depigmenting agents, antipigmenting agents, propigmenting agents, NO-synthase inhibitors, agents which stimulate the proliferation of fibroblasts and/or keratinocytes and/or the differentiation of keratinocytes, agents which act on microcirculation, agents which act on energy metabolism of the cells, healing agents, and mixtures thereof.

The composition according to the present invention may be in various forms, for example, suspensions, dispersions, solutions, gels, emulsions, such as oil-in-water (O/W), water-in-oil (W/O), and multiple, (e.g., W/O/W, polyol/O/W. and O/W/O) emulsions, creams, foams, sticks, dispersions of vesicles, for instance, of ionic and/or nonionic lipids, two-phase and multi-phase lotions, sprays, powders, and pastes. The composition may be anhydrous; for example, it can be an anhydrous paste or stick. The composition may also be a leave-in composition.

According to one embodiment, the composition according to the present invention may be in the form of an anhydrous composition such as a liquid or solid oily composition or a powdery composition.

According to another embodiment, the composition according to the present invention may be in the form of, for example, a compact powder, a lotion, a serum, a milk, a cream, a base foundation, an undercoat, a make-up base coat, a foundation, a face powder, cheek rouge, a lipstick, a lip cream, an eye shadow, an eyeliner, a loose powder, a concealer, a nail coat, mascara, a sunscreen and the like.

It is to be understood that a person skilled in the art can choose the appropriate presentation form, as well as its method of preparation, on the basis of his/her general knowledge, taking into account the nature of the constituents used, for example, their solubility in the vehicle, and the application envisaged for the composition.

### EXAMPLES

The present invention will be described in more detail by way of examples, which however should not be construed as limiting the scope of the present invention.

### Examples 1 and 2 and Comparative Examples 1 to 3

The components shown in Tables 1 and 2 were subjected to a hybridizer process using a Hybridizer equipped with a high speed rotor having a plurality of blades in a chamber in dry conditions, marketed by Nara Machinery Co., Ltd. in Japan to obtain a composite pigment.

In detail, for each of Example 1, Example 2 and Comparative Examples 1 to 3, the components shown in Tables 11 and 2 were mixed at the mixing ratio (the numerals in Tables 1 and 2 are based on parts by weight) shown in Tables 1 and 2 in a plastic bag by hand shaking for a short period of time. The mixture was put in the Hybridizer, and the rotor was revolved at 8,000 rpm (100 m/s linear velocity) for 5 minutes.

**Table 1**

| | Core | UV Filter |
|---|---|---|
| | NLK | TiO₂ |
| Ex. 1 | 100 | 40 |
| Ex. 2 | 100 | 100 |

**Table 2**

| | Core | | | UV Filter |
|---|---|---|---|---|
| | KSP | URE | NYL | TiO₂ |
| Comp. Ex. 1 | 100 | - | - | 40 |
| Comp. Ex. 2 | - | 100 | - | 40 |
| Comp. Ex. 3 | - | - | 100 | 100 |

- NLK:: Methylsilanol/silicate crosspolymer, in the form of a bowl-like particle, NLK 506 marketed by Takemoto Oil & Fat Co., Ltd. in Japan
- KSP:: Vinyl dimethicone/methicone silsesquioxane crosspolymer particle, KSP-100 marketed by Shin-etsu Chemical Co., Ltd. in Japan
- URE:: Crosslinked polyurethane particle, DAIMICBEAZ UCN-5150D NS CLEAR, marketed by Dainichiseika Color & Chemicals MFG. CO., LTD. in Japan
- NYL:: Nylon-12 particle marketed as Nylon SP-500 by Toray Industries Inc. in Japan
- TiO₂:: MT-100 TV marketed by Tayca Corporation in Japan

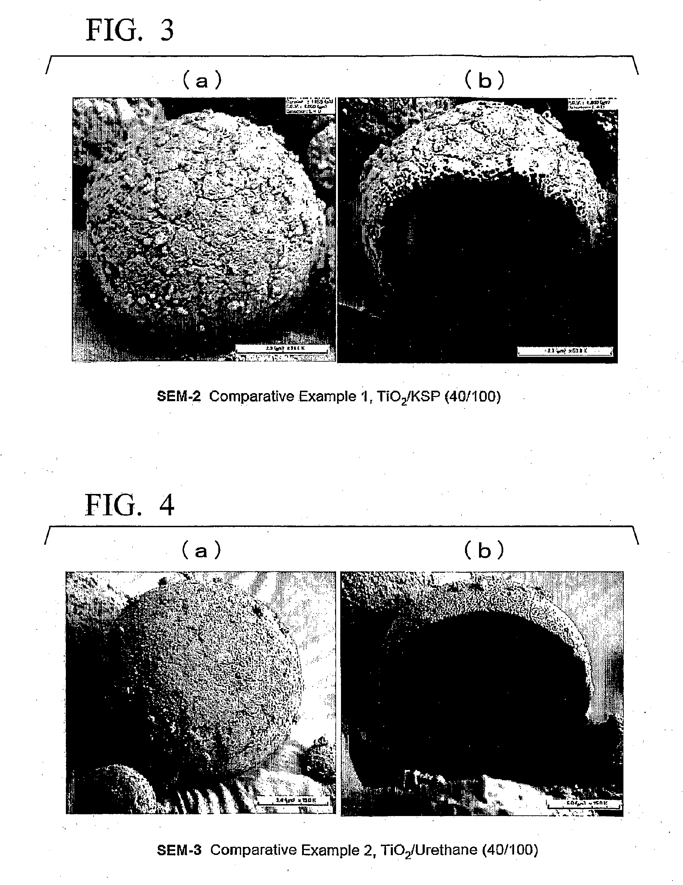

The obtained composite pigments according to Example 1 and Comparative Examples 1 to 3 were observed by FIB (focused ion beam)-SEM before and after cutting the composite pigment by a Ga ion beam. The SEM images of the composite pigment of Example 1 before and after the cutting are shown in Figure 2, as Figure 2(a) and Figure 2(b), respectively. The SEM images of the composite pigments of Comparative Examples 1 to 3 before and after the cutting are shown in Figures 3-5, as Figures 3-5(a) and Figures 3-5(b), respectively.

It is clear from Figure 2 that the composite pigment of Example 1 has a coating layer of TiO₂ which directly attaches onto the convex surface thereof. On the other hand, the composite pigments of Comparative Examples 1 to 3 have a coating layer of TiO₂ on the entire surface thereof as shown in Figures 3-5.

### Loose Powder

20 parts by weight of the composite pigment of Example 1 and 80 parts by weight of synthetic mica were mixed to prepare a loose powder. Likewise, 20 parts by weight of the composite pigment of Comparative Example 1 or 2 and 80 parts by weight of synthetic mica were mixed to prepare a loose powder as a control.

### [UVA and UVB Absorbance Determination]

Absorbance of UV waves was measured by use of a UV/VIS spectrophotometer type V-550 (JASCO, Japan) for the loose powders as follows.

6.0 mg of the loose powder was spread evenly by a finger on a 10 cm² tacky surface area of double faced tape attached on a transparent plastic sheet. The loose powder was covered with another transparent plastic sheet for sandwiching the powder sample. This test sheet was set in the V-550 sheet cell holder and the absorbance was measured from 260 nm to 400 nm. The averaged absorbance by the powder sample of 0.6 mg/cm² in the ranges of 260 nm to 320 nm and 320 nm to 400 nm were used for the values of the absorbance of UVB and UVA, respectively.

The results are shown in Table 3. In Table 3, Ex. 1 means a loose powder including the composite pigment of Example 1, and Comp. Ex. 1 and Comp. Ex. 2 mean a loose powder including the composite pigments of Comparative Examples 1 and 2, respectively.

**Table 3**

| | Ex. 1 | Comp. Ex. 1 | Comp. Ex. 2 |
|---|---|---|---|
| UVB absorbance | 0.88 | 0.73 | 0.51 |
| UVA absorbance | 0.45 | 0.36 | 0.24 |

As shown in Table 3, the loose powder including the composite pigment of Example 1 has a higher UV absorbance in both UVA, and UVB regions, than the loose powder including the composite pigment of Comparative Example 1 or 2.

### Powdery Foundation

A powdery foundation including the composite pigment of' Example 1 or a simple mixture of components corresponding to the composite pigment was prepared by mixing the composite pigment or the mixture with the components shown in Table 4.

**Table 4**

| | Wt% |
|---|---|
| Synthetic mica | 30.00 |
| Titanium oxide | 5.00 |
| Yellow iron oxide | 1.87 |
| Black iron oxide | 0.20 |
| Red iron oxide | 0.43 |
| Spherical silica | 5.00 |
| Talc | 37.50 |
| Mixture or Composite Pigment | 10.00 |
| Oil | 10.00 |
| Total | 100 |

In Table 4, "Mixture" means a simple mixture of the components shown in Table 1 for Example 1, and "Composite Pigment" means a composite pigment of Example 1.

### [Friction Coefficient Determination]

Friction Coefficient (MIU) was measured by use of a Tribomaster type TL201Sa (Trinity Lab., Inc., Japan) for the above powdery foundation as follows.

30 mg to 50 mg of the powdery foundation was deposited on a synthetic leather sheet fixed on the test stage of the Tribomaster by a double faced tape. A cubic aluminum probe with a 1 cm x 1 cm square head was also covered with the synthetic leather sheet and used for spreading the powdery foundation in a straight way for a distance of 2.0 cm at a speed of 1 cm/sec. The return strokes were repeated 5 times and the MIU was averaged from the 10 sets of MIU data in each forward and backward movement. The measurement was repeated three times, and the obtained results were averaged.

The results are shown in Table 5. In Table 5, [Mixture] means a powdery foundation including a simple mixture of the components shown in Table 1 for Example 1, and [Composite Pigment] means a powdery foundation including a composite pigment of Example 1.

**Table 5**

| | MIU [Composite Pigment] | MIU [Mixture] | Ratio (%) |
|---|---|---|---|
| Ex. 1 | 0.44 | 0.46 | 96 |

| | | | |
|---|---|---|---|
| Ratio(%):(Friction coefficient [Composite Pigment])/(Friction coefficient [Mixture])*100 | | | |

It is clear from Table 5 that the cosmetic comprising the composite pigment of Example 1 has a friction coefficient lower than that of the cosmetic comprising the mixture corresponding to the composite pigment.

Accordingly, cosmetics comprising the composite pigment according to the present invention can provide a smooth feeling on use which is better than that of conventional cosmetics comprising a simple mixture of the components of the composite pigment.

### Liquid Foundation

A liquid foundation including the composite pigment of Example 1 or a simple mixture of components corresponding to the composite pigment was prepared by mixing the composite pigment or the mixture with the components shown in Table 6.

**Table 6**

| | Wt% |
|---|---|
| Liquid base | 88.00 |
| Titanium oxide | 1.53 |
| Yellow iron oxide | 0.36 |
| Black iron oxide | 0.05 |
| Red iron oxide | 0.06 |
| Mixture or Composite Pigment | 10.00 |
| Total | 100 |

In Table 6, "Mixture" means a simple mixture of the components shown in Table 1 for Example 1, and "Composite Pigment" means a composite pigment of Example 1.

### [Friction Coefficient Determination]

Friction Coefficient (MIU) was measured by use of a Tribomaster type TL201Sa (Trinity Lab., Inc., Japan) for the above liquid foundation as follows.

A 15mg of the liquid foundation was deposited on the surface of a cubic aluminum probe with a 1 cm x 1 cm square head. The probe was covered with a synthetic leather sheet, and used for spreading the liquid foundation in a straight way for a distance of 2.0 cm at a speed of 1 cm/sec on the synthetic leather sheet fixed on the test stage of the Tribomaster by a double faced tape. The return strokes were repeated 30 times at 37°C and the MIU was averaged from the 60 sets of MIU data in each.

The results are shown in Table 7. In Table 7, [Mixture] means a liquid foundation including a simple mixture of the components shown in Table 1 for Example 1, and [Composite Pigment] means a liquid foundation including a composite pigment according to Example 1.

**Table 7**

| | MIU [Composite Pigment] | MIU [Mixture] | Ratio (%) |
|---|---|---|---|
| Ex. 1 | 0.33 | 0.35 | 94 |

| | | | |
|---|---|---|---|
| Ratio(%):(Friction coefficient [Composite Pigment])/(Friction coefficient [Mixture])*100 | | | |

It is clear from Table 7 that the cosmetic comprising the composite pigment of Example 1 has a friction coefficient lower than that of the cosmetic comprising the mixture corresponding to the composite pigment.

Accordingly, regardless of the type of cosmetics, cosmetics comprising the composite pigment according to the present invention can provide a smooth feeling on use which is better than that of conventional cosmetics comprising a mixture of the components of the composite pigment.

### Powdery Foundation

A powdery foundation including the composite pigment of Example 2 or Comparative Example 3 was prepared by mixing the composite pigment with the components shown in Table 8.

**Table 8**

| | Wt% |
|---|---|
| Synthetic mica treated with acrylates/dimethicone copolymer(95/5) | 30.00 |
| Titanium oxide | 5.00 |
| Yellow iron oxide | 1.87 |
| Black iron oxide | 0.20 |
| Red iron oxide | 0.43 |
| Spherical silica | 5.00 |
| Talc treated with isostearyl sebacate and vegetable amino acid | 37.50 |
| Composite Pigment | 10.00 |
| Oil | 10.00 |
| Total | 100 |

In Table 8, "Composite Pigment" means a composite pigment obtained in Example 2 or Comparative Example 3.

### [UVA and UVB Absorbance Determination]

Absorbance of UV waves was measured by use of a UV/VIS spectrophotometer type V-550 (JASCO, Japan) for the powdery foundations as described above.

The results are shown in Table 9. In Table 9, Ex. 2 means a powdery foundation including the composite pigment of Example 2, and Comp. Ex. 3 means a powdery foundation including the composite pigment of Comparative Example 3.

**Table 9**

| | Ex. 2 | Comp. Ex. 3 |
|---|---|---|
| UVB absorbance | 1.28 | 0.98 |
| UVA absorbance | 0.99 | 0.81 |

As shown in Table 9, the powdery foundation including the composite pigment of Example 2 has a higher UV absorbance in both UVA and UVB regions than the powdery foundation including the composite pigment of Comparative Example 3.

Accordingly, the cosmetic comprising the composite pigment according to the present invention can provide a better UV filtering effect than that of conventional cosmetics. This can be attributed to the good UV scattering property of the composite pigment used in Example 2.

### Mixture and Composite Pigment

A powdery foundation including 10 wt% of a simple mixture of the concave particle (NLK) and an inorganic UV filter (TiO₂) (weight ratio=100:100) corresponding to the composite pigment of Example 2 was prepared in accordance with the formulation shown in Table 8 using the mixture instead of the composite pigment of Example 2.

Next, a powdery foundation including 10 wt% of a mixture of a nylon particle (NYL) and an inorganic UV filter (TiO₂) (weight ratio=100:100) corresponding to the composite pigment of Comparative Example 3 was prepared in accordance with the formulation shown in Table 8 using the mixture instead of the composite pigment of Comparative Example 3.

### [UVA and UVB Absorbance Determination]

Absorbance of UV waves was measured by use of a UV/VIS spectrophotometer type V-550 (JASCO, Japan) for the powdery foundations as described above.

The results are shown in Tables 10 and 11. In Tables 10 and 11, Ex. 2 and Comp. Ex. 3 mean a powdery foundation including the composite pigment of Example 2 and Comparative Example 3, respectively, and Mix. Ex. 2 and Mix. Comp. Ex 3 mean a powdery foundation including a simple mixture of components corresponding to the composite pigment of Example 2 and Comparative Example 3, respectively.

**Table 10**

| | Ex. 2 | Mix. Ex. 2 |
|---|---|---|
| UVB absorbance | 1.28 | 1.36 |
| UVA absorbance | 0.99 | 0.94 |

**Table 11**

| | Comp. Ex. 3 | Mix. Comp. Ex. 3 |
|---|---|---|
| UVB absorbance | 0.98 | 1.51 |
| UVA absorbance | 0.81 | 1.08 |

As shown in Tables 10 and 11, the powdery foundation including the composite pigment of Example 2 has a comparable UV absorbance with that of the powdery foundation including a simple mixture of components corresponding to the composite pigment. On the other hand, the powdery foundation including the composite pigment of Comparative Example 3 has an inferior UV absorbance to that of the powdery foundation including a simple mixture of components corresponding to the composite pigment.

Accordingly, it is clear that the UV filtering effects of the composite pigment according to the present invention do not deteriorate but those of the conventional composite pigment deteriorate, as compared to a simple mixture of components corresponding to the composite pigment. This can be attributed to the fact that the UV filter (TiO₂) particles are compacted densely on the core particle in the composite pigment according to Comparative Example 3, which makes the apparent size of the UV filter large to lower the UV scattering property of the UV filter.

### Examples 3 and 4

The components shown in Table 12 were subjected to a hybridizer process using a Hybridizer equipped with a high speed rotor having a plurality of blades in a chamber in dry conditions, marketed by Nara Machinery Co., Ltd. in Japan to obtain a composite pigment.

In detail, for each of Examples 3 and 4, the components shown in Table 12 were mixed at the mixing ratio (the numerals in Table 12 are based on parts by weight) shown in Table 12 in a plastic bag by hand shaking for a short period of time. The mixture was put in the Hybridizer, and the rotor was revolved at 8,000 rpm (100 m/s linear velocity) for 5 minutes.

**Table 12**

| | Core | UV Filter | | |
|---|---|---|---|---|
| | NLK | TiO₂ | OMC | Mexoryl |
| Ex. 3 | 100 | 40 | 10 | - |
| Ex. 4 | 100 | 40 | - | 10 |

- NLK:: A bowl-like particle of methylsilanol/silicate crosspolymer, NLK 506 marketed by Takemoto Oil & Fat Co., Ltd. in Japan
- TiO₂:: MT-100 TV marketed by Tayca Corporation in Japan
- OMC:: Ethylhexyl methoxycinnamate
- Mexoryl:: Drometrizole trisiloxane

### Mixture and "Composite Pigment

A powdery foundation including the composite pigment of Example 3 or Example 4 was prepared by mixing the composite pigment with the components shown in Table 8.

On the other hand, a powdery foundation including 10 wt% of a mixture of a concave particle (NLK), an inorganic UV filter (TiO₂) and a liquid organic UV filter (OMC) (weight ratio=100:40:10) or a mixture of a concave particle (NLK), an inorganic UV filter (TiO₂) and a solid organic UV filter (Mexoryl) (weight ratio=100:40:10) was prepared in accordance with the formulation shown in Table 8 using the mixture instead of the composite pigment of Example 3 or 4, respectively.

### [UVA and UVB Absorbance Determination]

Absorbance of UV waves was measured by use of a UV/VIS spectrophotometer type V-550 (JASCO, Japan) for the powdery foundation as described above.

The results are shown in Tables 13 and 14. In Tables 13 and 14, Ex. 3 and Ex. 4 mean a powdery foundation including the composite pigment of Examples 3 and 4, respectively, and Mix. Ex. 3 and Mix. Ex. 4 mean a powdery foundation including a simple mixture of components corresponding to the composite pigment of Example 3 and 4, respectively.

**Table 13**

| | Ex. 3 | Mix. Ex. 3 |
|---|---|---|
| UVB absorbance | 1.64 | 1.67 |
| UVA absorbance | 1.04 | 1.04 |

**Table 14**

| | Ex. 4 | Mix. Ex. 4 |
|---|---|---|
| UVB absorbance | 1.41 | 1.49 |
| UVA absorbance | 1.11 | 1.13 |

As shown in Tables 13 and 14, the powdery foundation including the composite pigment of Example 3 or 4 has a comparable UV absorbance with that of the powdery foundation including a simple mixture of components corresponding to the composite pigment.

Accordingly, it is clear that the UV filtering effects of the composite pigment according to the present invention do not deteriorate as compared to a simple mixture of components constituting the composite pigment.

### Examples 5 and 6

Example 1 was repeated to obtain a composite pigment for Examples 5 and 6 except that the components shown in Table 15 (the numerals in Table 15 are based on parts by weight) were used for Examples 5 and 6.

**Table 15**

| | Core | Shell | |
|---|---|---|---|
| | NLK | TiO₂ | Red |
| Ex. 5 | 100 | - | 10 |
| Ex. 6 | 100 | 4 | 10 |

- NLK:: Methylsilanol/silicate crosspolymer in the form of a bowl-like particle, NLK 506 marketed by Takemoto Oil & Fat Co., Ltd. in Japan
- TiO₂:: MT-100 TV marketed by Tayca Corporation in Japan
- Red:: Red 202

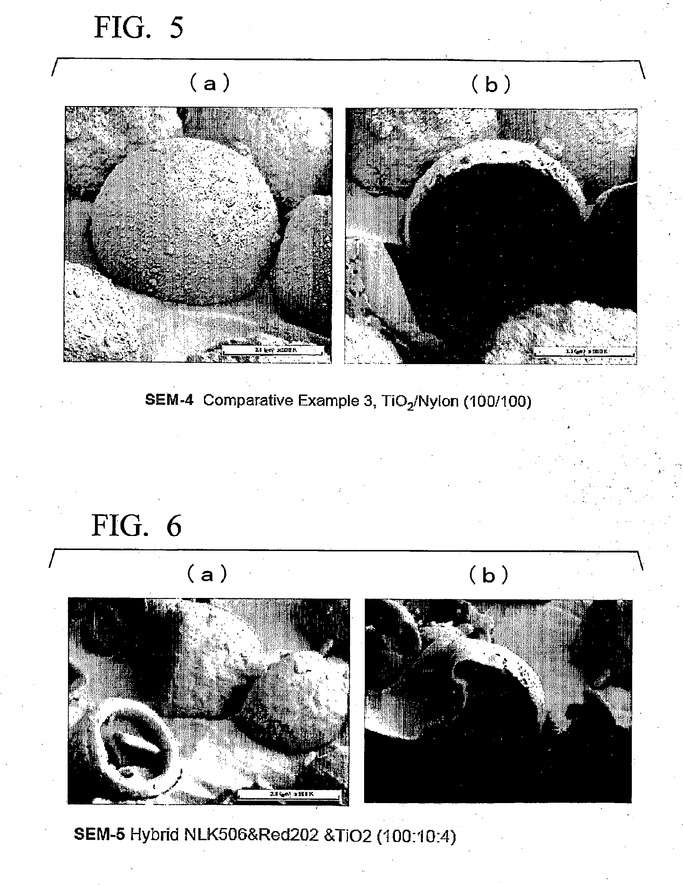

The obtained composite pigment according to Example 6 was observed by FIB (focused ion beam)-SEM before and after cutting the composite pigment by a Ga ion beam. The SEM images of the composite pigment of Example 6 before and after the cutting are shown in Figure 6, as Figure 6(a) and Figure 6(b), respectively.

It is clear from Figure 6 that the composite pigment of Example 6 has a coating layer of a UV filter (TiO₂) and a coloring pigment (Red 202) which directly attaches onto the convex surface thereof.

### Lipstick

A lipstick was prepared by mixing a base, the components of which are shown in Table 16, with the composite pigment according to Examples 5 and 6 or a simple mixture of components corresponding to the composite pigment of Examples 5 and 6 such that the coloring pigment (Red 202) corresponds to 0.1wt% of the lipstick, with a tricylinder roller at 90°C.

**Table 16**

| | Wt% |
|---|---|
| DIGLYCEROL ISOSTEARATE 3%, DIGLYCEROL DIISOSTEARATE 90%, DIGLYCEROL TRIISOSTEARATE 5%, ISOSTEARIC ACID 2% ISOSTEARIC ACID 2% | 19.05 |
| DI-MERATE DE DI-ISO-PROPYLE | 17.65 |
| HYDROGENATED POLYISOBUTENE | 14.29 |
| ISOPARAFFINE (6-8 MOLES D'ISOBUTYLENE) HYDROGENEE | 13.93 |
| ESTERS D'ACIDES GRAS VEGETAUX, ISO-STEARIQUE, ADIPIQUE DE GLYCERYLE | 11.91 |
| PURE JOJOBA OIL | 11.19 |
| LIPEX 102 SHEA BUTTER | 7.14 |
| VP/HEXADECENE COPOLYMER | 4.77 |
| DITERTIOBUTYL 4-HYDROXYTOLUENE | 0.07 |
| Total | 100 |

### [Color Determination]

Color (L*,a*,b*) of each of the above lipsticks was measured by using a DATACOLOR 600 (Applied Color Systems Inc., US) as follows.

2.5g of the lipstick was poured into an aluminum pan (25mm(L)*23mm(W)*4mm(D)) and cooled to solidify the paste. The L*, a* and b* of the test sample were measured from the top of the paste.

The results are shown in Tables 17-19. In Tables 17-19, [Mixture] means a lipstick including a simple mixture of the components shown in Table 15, and [Composite Pigment] means a lipstick including a composite pigment obtained by the hybridizer process for the components shown in Table 15.

**Table 17**

| | L* | |
|---|---|---|
| | [Composite Pigment] | [Mixture] |
| Ex. 5 | 43.24 | 42.84 |
| Ex. 6 | 43.21 | 43.13 |

**Table 18**

| | a* | |
|---|---|---|
| | [Composite Pigment] | [Mixture] |
| Ex. 5 | 51.07 | 45.77 |
| Ex. 6 | 51.02 | 46.14 |

**Table 19**

| | b* | |
|---|---|---|
| | [Composite Pigment] | [Mixture] |
| Ex. 5 | 18.91 | 13.57 |
| Ex. 6 | 20.29 | 12.13 |

It is clear from Tables 17-19 that the cosmetics comprising the composite pigments of Examples 5 and 6 have higher a* and b* values as compared to those comprising a simple mixture of components corresponding to the composite pigments.

Accordingly, cosmetics comprising the composite pigment according to the present invention can provide a better color with higher chroma compared to conventional cosmetics comprising a simple mixture of components corresponding to the composite pigment.

### [Visible Light Absorbance Determination]

Absorbance of visible light was measured by use of a UV/VIS spectrophotometer type V-550 (JASCO, Japan) for the lipstick as described in the above [UVA and UVB Absorbance Determination], providing that the absorbance in the wavelength of 400-700nm was measured instead of the UVA and UVB regions.

The results are shown in Table 20. In Table 20, [Mixture] means a lipstick including a simple mixture of the components shown in Table 15, and [Composite Pigment] means a lipstick including a composite pigment obtained by the hybridizer process for the components shown in Table 15.

**Table 20**

| | Visible Light Absorption | |
|---|---|---|
| | [Composite Pigment] | [Mixture] |
| Ex. 5 | 0.69 | 0.82 |
| Ex. 6 | 0.73 | 0.95 |

As shown in Table 20, the lipsticks including the composite pigments of Examples 5 and 6 have a lower absorbance in visible light than the lipsticks including a simple mixture of components corresponding to the composite pigments.

Accordingly, cosmetics comprising the composite pigment according to the present invention can provide a more transparent appearance compared to conventional cosmetics comprising a simple mixture of components corresponding to the composite pigment.

### Example 7

The components shown in Table 21 were subjected to a hybridizer process using a Hybridizer equipped with a high speed rotor having a plurality of blades in a chamber in dry conditions, marketed by Nara Machinery Co., Ltd. in Japan to obtain a composite pigment.

In detail, for Example 7, the components shown in Table 21 were mixed at the mixing ratio (the numerals in Table 21 are based on parts by weight) shown in Table 21 in a plastic bag by hand shaking for a short period of time. The mixture was put in the Hybridizer, and the rotor was revolved at 8,000 rpm (100 m/s linear velocity) for 5 minutes.

**Table 21**

| | Core | UV Filter | | |
|---|---|---|---|---|
| | NLK* | TiO₂ | OMC | Mexoryl |
| Ex. 7 | 100 | 40 | 10 | 10 |

- NLK:: Methylsilanol/silicate crosspolymer in the form of a rugby ball-like particle, NLK 602 marketed by Takemoto Oil & Fat Co., Ltd. in Japan
- TiO₂:: MT-100 TV marketed by Tayca Corporation in Japan
- OMC:: Ethylhexyl methoxycinnamate
- Mexoryl:: Drometrizole trisiloxane

### Mixture and Composite Pigment

A powdery foundation including the composite pigment of Example 7 was prepared by mixing the composite pigment with the components shown in Table 8.

On the other hand, a powdery foundation including 10wt% of a mixture of a concave particle (NLK*), an inorganic UV filter (TiO₂), a liquid organic UV filter (OMC) and a solid organic UV filter (Mexoryl) (weight ratio=100:40:10:10) was prepared in accordance with the formulation shown in Table 8 using the mixture instead of the composite pigment of Example 7.

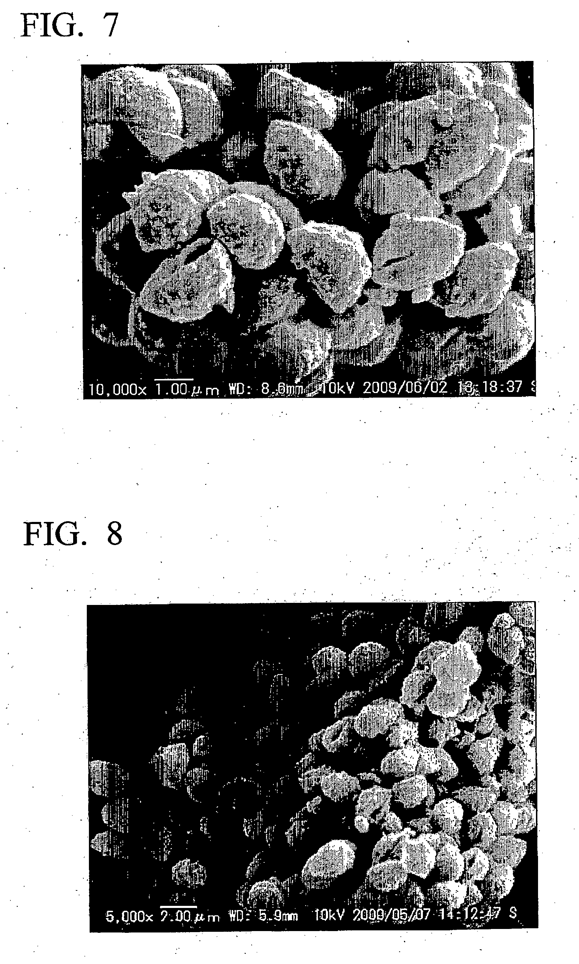

The SEM images of the composite pigment of Example 7 and the above mixture are shown in Figures 7 and 8, respectively.

It is clear from Figure 7 that the concave particle (NLK*) used in Example 7 corresponds to a closed or crushed type of the concave particle (NLK) used in Examples 1 to 6 and that a concave therein which connects to the outer environment is present therein.

### [UVA and UVB Absorbance Determination]

Absorbance of UV waves was measured by use of a UV/VIS spectrophotometer type V-550 (JASCO, Japan) for the powdery foundation as described above.

The results are shown in Table 22. In Table 22, Ex. 7 means a powdery foundation including the composite pigment of Example 7, and Mix. Ex. 7 means a powdery foundation including a simple mixture of components corresponding to the composite pigment of Example 7.

**Table 22**

| | Ex. 7 | Mix. Ex. 7 |
|---|---|---|
| UVB absorbance | 1.30 | 1.27 |
| UVA absorbance | 0.86 | 0.70 |

As shown in Table 22, the powdery foundation including the composite pigment of Example 7 has a higher UV absorbance than the powdery foundation including a simple mixture of a core particle and a UV filter corresponding to the composite pigment.

Accordingly, it is clear that the UV filtering effects of the composite pigment according to the present invention do not deteriorate, rather are enhanced, as compared to a simple mixture of components constituting the composite pigment.

### [Color Determination]

Color (L*) of each of the above foundations was measured by using a DATACOLOR 600 (Applied Color Systems Inc., US) as described above.

The results are shown in Table 23. In Table 23, [Mixture] means a foundation including a simple mixture of the components shown in Table 21, and [Composite Pigment] means a lipstick including a composite pigment obtained by the hybridizer process for the components shown in Table 21.

**Table 23**

| | L* | |
|---|---|---|
| | [Composite Pigment] | [Mixture] |
| Ex. 7 | 0.82 | 0.79 |

It is clear from Table 23 that the cosmetic comprising the composite pigment of Example 7 has a higher L* value as compared to that comprising a simple mixture of components corresponding to the composite pigment.

Accordingly, cosmetics comprising the composite pigment according to the present invention can provide a better covering effect compared to conventional cosmetics comprising a simple mixture of components corresponding to the composite pigment.

## Claims

1. A composite pigment comprising a substrate with a general concave shape, wherein the substrate with a general concave shape defines an inner concave surface defining an inner peripheral edge and an outer convex surface having an outer peripheral edge, wherein the average diameter of said inner peripheral edge ranges from 0.1 to 20µm, the average diameter of said outer peripheral edge ranges from 0.2 to 30µm, and the average concavity of the inner concave surface, as measured along an axis perpendicular to said inner peripheral edge, ranges from 0.1 to 20 µm, said substrate being at least in part covered by at least one layer comprising at least one UV filter and/or at least one coloring pigment, wherein the substrate with a general concave shape comprises poly (meth) acrylate, an organosilicone material, or mixtures thereof, wherein the at least one UV filter comprises an organic filter selected from the group consisting of anthranilic derivatives; dibenzoylmethane derivatives; cinnamic derivatives; salicylic derivatives; camphor derivatives; benzophenone derivatives; [beta], [beta]-diphenylacrylate derivatives; triazine derivatives; benzotriazole derivatives; benzalmalonate derivatives; benzimidazole derivatives; imidazoline derivatives; bis-benzoazolyl derivatives; p-aminobenzoic acid (PABA) and derivatives thereof; methylenebis (hydroxyphenylbenzo-triazole) derivatives; benzoxazole derivatives; screening polymers and screening silicones; dimers derived from [alpha]-alkylstyrene; 4,4- diarylbutadiene derivatives; octocrylene and derivatives thereof, guaiazulene and derivatives thereof, rutin and derivatives thereof, flavonoids, biflavonoids, oryzanol and derivatives thereof, quinic acid and derivatives thereof, phenols, retinol, cysteine, aromatic amino acid, peptides having an aromatic amino acid residue, and mixtures thereof, or an inorganic UV filter selected from the group consisting of silicon carbide, metal oxides which may or may not be coated, and mixtures thereof, and wherein the at least one coloring pigment is chosen from titanium dioxide, zirconium oxide, cerium oxide, zinc oxides, iron oxides, chromium oxide, manganese violet, ultramarine blue, chromium hydrate, ferric blue, aluminum powder, copper powder, carbon black, pigments of D&C type, lakes, pearlescent pigments, and mixtures thereof.

2. The composite pigment according to Claim 1, wherein the substrate with a general concave shape has a mean diameter ranging from 0. 1 µm to 30µm.

3. The composite pigment according to Claim 1 or 2, wherein the substrate defines an inner concave surface and an outer convex surface, opposite said inner concave surface, with the at least one layer essentially covering said outer convex surface.

4. The composite pigment according to any one of Claims 1 to 3, wherein the substrate defines an inner concave surface and an outer convex surface, opposite said inner concave surface, with the at least one layer essentially covering said inner concave surface.

5. The composite pigment according to any one of Claims 1 to 4, wherein the substrate with a general concave shape is in the form of a portion of a hollow sphere.

6. The composite pigment according to any one of Claims 1 to 5, wherein the at least one UV filter is organic or inorganic.

7. The composite pigment according to any one of Claims 1 to 6, wherein the weight ratio of said substrate with a general concave shape to the UV filter (s) and/or coloring pigment (s) is 100:1 to 100:500.

8. A method for preparing a composite pigment according to any one of Claims 1 to 7, comprising a step of subjecting a substrate with a general concave shape and at least one UV filter and/or at least one coloring pigment to a hybridizer process.

9. A cosmetic composition comprising a composite pigment according to any one of Claims 1 to 7.

## Patentansprüche

1. Verbundpigment, umfassend ein Substrat mit einer im Allgemeinen konkaven Gestalt, wobei das Substrat mit einer im Allgemeinen konkaven Gestalt eine innere konkave Fläche definiert, die wiederum eine innere periphere Kante und eine äußere konvexe Fläche mit einer äußeren peripheren Kante definiert, wobei der durchschnittliche Durchmesser der genannten inneren peripheren Kante im Bereich von 0,1 bis 20 µm liegt, der durchschnittliche Durchmesser der genannten äußeren peripheren Kante im Bereich von 0,2 bis 30 µm liegt und die durchschnittliche Konkavität der inneren konkaven Fläche, wie entlang einer senkrecht zur genannten inneren peripheren Kante liegenden Achse gemessen, im Bereich von 0,1 bis 20 µm liegt, wobei das genannte Substrat wenigstens teilweise von einer ersten Schicht bedeckt ist, die wenigstens einen UV-Filter und/oder wenigstens ein Farbpigment umfasst, wobei das Substrat mit einer im Allgemeinen konkaven Gestalt Poly(meth)acrylat, ein Organosiloxanmaterial oder Mischungen davon umfasst, wobei der wenigstens eine UV-Filter einen organischen Filter umfasst, der ausgewählt ist aus der Gruppe bestehend aus Anthranilderivaten; Dibenzoylmethanderivaten; Zimtderivaten; Salizylderivaten; Campherderivaten; Benzphenonderivaten; [Beta]; [Beta]-Diphenylacrylatderivaten; Triazinderivaten; Benztriazolderivaten; Benzalmalonatderivaten; Benzimidazolderivaten; Imidazolinderivaten; Bis-Benzazolylderivaten; p-Aminobenzoesäure (PABA) und den Derivaten davon; Methylenbis- (Hydroxyphenylbenztriazol-) Derivaten; Benzoxazolderivaten; Screening-Polymeren und Screening-Silikonen; den von [Alpha]-Alkylstyrol abgeleiteten Dimeren; 4,4-Diarylbutadienderivaten; Octocrylen und den Derivaten davon, Guaiazulen und den Derivaten davon, Rutin und den Derivaten davon, Flavonoiden, Biflavonoiden, Oryzanol und den Derivaten davon, Chinasäure und den Derivaten davon, Phenolen, Retinol, Cystein, aromatischer Aminosäure, Peptiden mit einem aromatischen Aminosäurerest sowie den Mischungen davon, oder einen anorganischen UV-Filter, der ausgewählt ist aus der Gruppe bestehend aus Siliziumkarbid, Metalloxiden, die beschichtet sein können oder nicht, sowie Mischungen davon und wobei das wenigstens eine Farbpigment ausgewählt ist aus Titandioxid, Zirkondioxid, Ceroxid, Zinkoxiden, Eisenoxiden, Chromoxiden, Manganviolett, Ultramarinblau, Chromhydrat, Eisenblau, Aluminiumpulver, Kupferpulver, Kohlenschwarz, den Pigmenten der D & C Art, Laken, Perlglanzpigmenten und den Mischungen davon.

2. Verbundpigment nach Anspruch 1, wobei das Substrat mit einer im Allgemeinen konkaven Gestalt einen mittleren Durchmesser im Bereich von 0,1 µm bis 30 µm aufweist.

3. Verbundpigment nach Anspruch 1 oder 2, wobei das Substrat eine innere konkave Fläche und eine äußere konvexe Fläche gegenüber der genannten inneren konkaven Fläche definiert, wobei die wenigstens eine Schicht im Wesentlichen die genannte äußere konvexe Fläche bedeckt.

4. Verbundpigment nach einem der Ansprüche 1 bis 3, wobei das Substrat eine innere konkave Fläche und eine äußere konvexe Fläche gegenüber der genannten inneren konkaven Fläche definiert, wobei die wenigstens eine Schicht im Wesentlichen die genannte innere konkave Fläche bedeckt.

5. Verbundpigment nach einem der Ansprüche 1 bis 4, wobei das Substrat mit einer im Allgemeinen konkaven Gestalt in Form eines Anteils einer Hohlkugel besteht.

6. Verbundpigment nach einem der Ansprüche 1 bis 5, wobei der wenigstens eine UV-Filter organisch oder anorganisch ist.

7. Verbundpigment nach einem der Ansprüche 1 bis 6, wobei das Gewichtsverhältnis des genannten Substrats mit einer im Allgemeinen konkaven Gestalt zum/zu den UV-Filter(n) und/oder dem/den Farbpigment(en) 100:1 bis 100:500 beträgt.

8. Verfahren zur Herstellung eines Verbundpigments nach einem der Ansprüche 1 bis 7, umfassend einen Schritt des Aussetzens eines Substrats mit einer im Allgemeinen konkaven Gestalt und wenigstens eines UV-Filters und/oder wenigstens eines Farbpigments an einen Hybridisierungsprozess.

9. Kosmetische Zusammensetzung, umfassend ein Verbundpigment nach einem der Ansprüche 1 bis 7.

## Revendications

1. Pigment composite comprenant un substrat ayant une forme générale concave, dans lequel le substrat ayant une forme générale concave définit une surface concave interne définissant un bord périphérique interne et une surface convexe externe ayant un bord périphérique externe, dans lequel le diamètre moyen dudit bord périphérique interne est dans la plage de 0,1 à 20 µm, le diamètre moyen dudit bord périphérique externe est dans la plage de 0,2 à 30 µm, et la concavité moyenne de la surface concave interne, telle que mesurée le long d'un axe perpendiculaire audit bord périphérique interne, est dans la plage de 0,1 à 20 µm, ledit substrat étant au moins partiellement recouvert par au moins une couche comprenant au moins un filtre UV et/ou au moins un pigment colorant, dans lequel le substrat ayant une forme générale concave comprend du poly(méth)acrylate, un matériau d'organosilicone, ou des mélanges de ceux-ci, l'au moins un filtre UV comprenant un filtre organique choisi dans le groupe constitué de dérivés anthraniliques ; dérivés de dibenzoylméthane ; dérivés cinnamiques ; dérivés salicyliques ; dérivés de camphre ; dérivés de benzophénone ; dérivés de [bêta], [bêta]-diphénylacrylate ; dérivés de triazine ; dérivés de benzotriazole ; dérivés de benzalmalonate ; dérivés de benzimidazole ; dérivés d'imidazoline ; dérivés de bis-benzoazolyle ; acide p-aminobenzoïque (PABA) et dérivés de celui-ci ; dérivés de méthylènebis(hydroxyphénylbenzotriazole) ; dérivés de benzoxazole ; polymères de sélection et silicones de sélection ; dimères dérivés de [alpha]-alkylstyrène ; dérivés de 4,4-diarylbutadiène ; octocrylène et dérivés de celui-ci, guaïazulène et dérivés de celui-ci, rutine et dérivés de celle-ci, flavonoïdes, biflavonoïdes, oryzanol et dérivés de celui-ci, acide quinique et dérivés de celui-ci, phénols, rétinol, cystéine, acide aminé aromatique, peptides ayant un résidu d'acide aminé aromatique, et des mélanges de ceux-ci, ou un filtre UV inorganique choisi dans le groupe constitué du carbure de silicium, d'oxydes de métal qui peuvent être ou non revêtus, et des mélanges de ceux-ci, et l'au moins un pigment colorant étant choisi parmi le dioxyde de titane, l'oxyde de zirconium, l'oxyde de cérium, les oxydes de zinc, les oxydes de fer, l'oxyde de chrome, le violet de manganèse, le bleu outremer, l'hydrate de chrome, le bleu ferrique, la poudre d'aluminium, la poudre de cuivre, le noir de carbone, les pigments de type D & C, les laques, les pigments nacrés, et des mélanges de ceux-ci.

2. Pigment composite selon la revendication 1, dans lequel le substrat avec une forme concave générale a un diamètre moyen dans la plage de 0,1 µm à 30 µm.

3. Pigment composite selon la revendication 1 ou 2, dans lequel le substrat définit une surface concave interne et une surface convexe externe, opposée à ladite surface concave interne, l'au moins une couche recouvrant essentiellement ladite surface convexe externe.

4. Pigment composite selon l'une quelconque des revendications 1 à 3, dans lequel le substrat définit une surface concave interne et une surface convexe externe, opposée à ladite surface concave interne, l'au moins une couche recouvrant essentiellement ladite surface concave interne.

5. Pigment composite selon l'une quelconque des revendications 1 à 4, dans lequel le substrat ayant une forme générale concave est sous la forme d'une partie d'une sphère creuse.

6. Pigment composite selon l'une quelconque des revendications 1 à 5, dans lequel l'au moins un filtre UV est organique ou inorganique.

7. Pigment composite selon l'une quelconque des revendications 1 à 6, dans lequel le rapport en poids dudit substrat ayant une forme générale concave au(x) filtre(s) UV et/ou pigment(s) colorant(s) est de 100:1 à 100:500.

8. Procédé de préparation d'un pigment composite selon l'une quelconque des revendications 1 à 7, comprenant une étape de soumission d'un substrat ayant une forme générale concave et au moins un filtre UV et/ou au moins un pigment colorant à un processus d'hybridation.

9. Composition cosmétique comprenant un pigment composite selon l'une quelconque des revendications 1 à 7.
